(19) Europäisches Patentamt
European Patent Office
Office européen des brevets

(11) EP 4 667 577 A1

(12) EUROPEAN PATENT APPLICATION
published in accordance with Art. 153(4) EPC

(43) Date of publication:
**24.12.2025 Bulletin 2025/52**

(21) Application number: **24756871.0**

(22) Date of filing: **13.02.2024**

(51) International Patent Classification (IPC):
***C12N 15/864*** (2006.01) ***C12N 15/34*** (2006.01)
***C12N 15/35*** (2006.01) ***C12N 15/88*** (2006.01)

(52) Cooperative Patent Classification (CPC):
**C07K 14/01; C07K 14/015; C12N 15/864; C12N 15/88**

(86) International application number:
**PCT/JP2024/004838**

(87) International publication number:
**WO 2024/172027 (22.08.2024 Gazette 2024/34)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **17.02.2023 JP 2023023545**

(71) Applicant: **Synplogen Co., Ltd.**
**Kobe-shi, Hyogo 650-0047 (JP)**

(72) Inventors:
- **NISHIMURA, Yuya**
  **Kobe-shi, Hyogo 650-0047 (JP)**
- **SAITO, Shunsuke**
  **Kobe-shi, Hyogo 650-0047 (JP)**

(74) Representative: **Vossius & Partner Patentanwälte Rechtsanwälte mbB**
**Siebertstraße 3**
**81675 München (DE)**

Remarks:
The complete document including Reference Table(s) and the Sequence Listing(s) can be downloaded from the EPO website

(54) **ITR-REP GENE COMPLEX**

(57) The present disclosure provides plasmid DNA or a DNA/cationic substance complex containing a Rep and an ITR and exhibiting high AAV vector productivity. More specifically, in one exemplary aspect, the present disclosure provides a nucleic acid containing a Rep gene derived from an adeno-associated virus (AAV) of a first serotype, and an inverted terminal repeat (ITR) sequence derived from an AAV of a second serotype different from the first serotype and having a ΔG value in a range of -65 to -95 kcal/mol.

EP 4 667 577 A1

## Description

[Technical Field]

The present disclosure provides plasmid DNA or a DNA/cationic substance complex containing a Rep and an ITR and exhibiting high AAV vector productivity.

[Background Art]

When an AAV vector is produced, the ITR and Rep proteins interact with each other and encapsidate DNA in capsid proteins. In recent years, with the expansion of applicable diseases for which AAV vectors are used and an increase in production costs, there is a demand for a technique that can increase the yield of AAV vectors.

Summary of Invention

[Solution to Problem]

The present inventors have found certaincombination of an ITR and a Rep, other than the ITR2/Rep2 combination, that exhibits high AAV vector productivity. Accordingly, the present disclosure provides plasmid DNA or a DNA/cationic substance complex containing a combination of a Rep and an ITR that exhibits high AAV vector productivity.

Accordingly, the present disclosure provides the following.

(Item 1) A nucleic acid containing:

a Rep gene derived from an adeno-associated virus (AAV) of a first serotype; and
an inverted terminal repeat (ITR) sequence derived from an AAV of a second serotype different from the first serotype and having a $\Delta G$ value in a range of -65 to -95 kcal/mol.

(Item 2) The nucleic acid according to the above item, in which the Rep gene is derived from an AAV of serotype 2, and the ITR sequence has a $\Delta G$ value in a range of -75 to -95 kcal/mol.
(Item 3) The nucleic acid according to any one of the above items, in which the ITR sequence has a $\Delta G$ value in a range of -80 to -88 kcal/mol.
(Item 4) The nucleic acid according to any one of the above items, in which the ITR sequence is derived from an AAV of serotype 1, 6, or 7.
(Item 4A) The nucleic acid according to any one of the above items, in which the Rep gene is selected from the group consisting of Rep1/6, Rep2, Rep3/4, Rep12, Rep5, Rep8, Rep7/9, and Rep10/11.
(Item 4B) The nucleic acid according to any one of the above items, in which the Rep gene encodes an amino acid sequence having 90% or more identity to at least one of Rep3, Rep1, or Rep7.
(Item 5) The nucleic acid according to any one of the above items, in which the Rep gene is derived from an AAV of serotype 1, 2, 5, 7, or 10, and the ITR sequence has a $\Delta G$ value in a range of -75 to -95 kcal/mol.
(Item 6) The nucleic acid according to any one of the above items, in which the ITR sequence has a $\Delta G$ value in a range of -80 to -90 kcal/mol.
(Item 7) The nucleic acid according to any one of the above items, in which the ITR sequence is derived from an AAV of any one of serotypes 1 to 7.
(Item 8) The nucleic acid according to any one of the above items, further containing a Cap gene derived from an AAV.
(Item 9) The nucleic acid according to any one of the above items, further containing a Cap gene derived from an AAV of any one of serotypes 1 to 10.
(Item 10) The nucleic acid according to any one of the above items, in which the Rep gene, the ITR sequence, and a Cap gene derived from an AAV are contained on the same nucleic acid molecule or nucleic acid duplex.
(Item 11) The nucleic acid according to any one of the above items, further containing a helper gene derived from an adenovirus (AdV).
(Item 12) The nucleic acid according to any one of the above items, in which a combination of the Rep gene and the ITR sequence is a combination of the Rep gene and the ITR sequence, which has an ability to produce a viral vector with a titer [GC/mL] that is twice or more than that of a combination of the said Rep gene and ITR-5, when measured by qPCR targeting a CMV promoter or EGFP.
(Item 13) The nucleic acid according to any one of the above items, in which the nucleic acid is a plasmid.
(Item 14) The nucleic acid according to any one of the above items, in which the nucleic acid forms a complex with a cationic substance.

(Item 15) The nucleic acid according to any one of the above items, in which the cationic substance contains a polymer or a liposome.

(Item 16) A composition for producing a viral vector, the composition containing the nucleic acid according to any one of the above items.

(Item 17) A method for synthesizing the nucleic acid according to any one of the above items, the method including a step of combining a nucleic acid containing the Rep gene and a nucleic acid containing the ITR.

(Item 18) A method for producing a viral vector, the method including:

a step of introducing the nucleic acid according to any one of the above items or the composition according to any one of the above items into a producer cell; and

a step of forming a viral vector in the producer cell.

(Item 19) The method according to any one of the above items, in which at least a part of the nucleic acid is integrated into a chromosome of the producer cell.

(Item 20) A producer cell or a viral vector produced by the method according to any one of the above items.

In the present disclosure, it is intended that the one or the plurality of features can be provided in further combination in addition to the specified combination. Still further embodiments and advantages of the present disclosure will be appreciated by those skilled in the art upon reading and understanding the following detailed description, if necessary.

Advantageous Effects of Invention

The present disclosure provides plasmid DNA or a DNA/cationic substance complex containing a combination of a Rep and an ITR that exhibits high AAV vector productivity.

Brief Description of Drawings

[FIG. 1] FIG. 1 illustrates a schematic diagram of 56 patterns of constructs of 8 types of ITRs and 7 types of Reps.

[FIG. 2] FIG. 2 illustrates the measurement results of the constructs in FIG. 1 by qPCR and HPLC.

[FIG. 3] FIG. 3 illustrates a schematic diagram of 14 patterns of constructs of 7 types of ITRs, 1 type of Rep, and 2 types of Cap2.

[FIG. 4] FIG. 4 illustrates the measurement results of the constructs in FIG. 3 by qPCR and HPLC.

[FIG. 5] FIG. 5 illustrates graphs of $\Delta G$ of the 5' ITR and 3' ITR.

[Description of Embodiments]

Hereinafter, the present disclosure will be described while showing the best mode. Throughout the present specification, the expression of singular forms is to be understood as including the concept of plural forms unless otherwise stated. Therefore, the singular article (for example, "a", "an", "the", or the like in English) should be understood to include the concept of plural forms unless otherwise stated. In addition, the terms used herein are to be understood as being used in the sense commonly used in the art unless otherwise stated. Therefore, unless defined otherwise, all technical terms and scientific terms used herein have the same meaning as commonly understood by those skilled in the art to which the present disclosure pertains. In the case of conflict, the present specification (including definitions) will control.

Hereinafter, the definitions of the terms and/or basic technical concepts that are particularly used herein will be described as appropriate.

As used herein, the terms "polynucleotide" and "nucleic acid" are used to have the same meaning, and refer to a polymer of nucleotides of any length. As used herein, unless otherwise specified, the term "nucleic acid" refers to both a single nucleic acid molecule and a plurality of nucleic acid molecules, as well as both a single-stranded nucleic acid and a double-stranded nucleic acid. As used herein, unless otherwise specified, a description that a nucleic acid contains a plurality of elements includes embodiments in which the plurality of elements are present on the same nucleic acid molecule and embodiments in which the plurality of elements are present on different nucleic acid molecules, and includes embodiments in which the plurality of elements are present in the same double-stranded nucleic acid complex and embodiments in which the plurality of elements are present in different double-stranded nucleic acid complexes. Examples of the nucleic acid include DNA, RNA, cDNA, mRNA, rRNA, tRNA, microRNA (miRNA), and lncRNA. The term also includes a "polynucleotide derivative". The term "polynucleotide derivative" refers to a polynucleotide containing a nucleotide derivative or having an unusual bond between nucleotides. The term "nucleotide derivative" refers to a nucleotide having a structure different from a normal nucleotide used in natural DNA or RNA, and examples thereof include a locked nucleic acid (LNA), an ethylene nucleic acid such as a 2'-O,4'-C-ethylene bridged nucleic acid (ENA), other bridged nucleic acids (BNAs), a hexitol nucleic acid (HNA), an amido-bridged nucleic acid (AmNA), a morpholino nucleic acid, tricyclo-DNA (tcDNA), a polyether nucleic acid (see, for example, US 5,908,845), and a cyclohexene nucleic acid (CeNA). Examples of the unusual bond between nucleotides include a bond between oligonucleotides in which a phosphate diester bond is converted into a phosphorothioate bond, a bond between oligonucleotides in which a phosphate diester bond is converted into an N3'-P5' phosphoramidate bond, and a bond between oligonucleotides in which ribose and a phosphate diester bond are converted to peptide nucleic acid bonds.

Unless otherwise indicated, a particular nucleic acid sequence is also intended to encompass a conservatively modified variant (for example, condensed codon substituent) and complementary sequence thereof, as well as the sequence explicitly indicated. Specifically, the condensed codon substituent may be achieved by generating a sequence in which the third position of one or more selected (or all) codons is substituted with a mixed base and/or deoxyinosine residue. Examples of variants based on specific wild-type sequences such as adenovirus serotypes 1 to 52 and adeno-associated virus serotypes 1 to 12 include not only known variants (for example, rh10, DJ, DJ/8, PHP.eB, PHP.S, AAV2-retro, AAV2-QuadYF, AAV2.7m8, AAV6.2, rh.74, AAV2.5, AAV-TT, Anc80, and the like) but also nucleic acids having sequences having at least 70%, at least 80%, at least 85%, at least 90%, at least 95%, at least 96%, at least 97%, at least 98%, at least 99%, or at least 99.5% sequence identity to the original sequence.

As used herein, the term "gene" refers to a nucleic acid segment that performs a certain biological function. The biological function includes coding for polypeptides or proteins, coding for protein non-coding functional RNAs (rRNA, tRNA, microRNA (miRNA), lncRNA, and the like), regulating the production of polypeptides, proteins, or non-coding functional RNAs, specifically binding to specific proteins, and regulating cleavage or replication of nucleic acids. Therefore, as used herein, the gene contains a transcriptional and translational regulatory sequence such as a promoter, a terminator, an enhancer, an insulator, a silencer, an origin of replication, or an internal ribosome entry site, and a nucleic acid segment required for packaging into viral particles, in addition to a nucleic acid segment encoding a protein or a protein non-coding functional RNA. As used herein, the term "gene product" refers to an mRNA transcribed from a gene, a polypeptide or protein encoded by a gene, or a protein non-coding functional RNA. As used herein, the term "expression of a gene" refers to the production of a gene product. In particular, viral genes often encode multiple types of proteins of different lengths in a single nucleic acid region. As used herein, when at least one type of protein that may be produced from a gene is produced, the gene is referred to as being expressed.

As used herein, the term "adeno-associated virus" (AAV) refers to a linear single-stranded DNA virus of the genus *Dependoparvovirus* in the family *Parvoviridae,* and the viral particle has a diameter of 20 to 26 nm. Proliferation of AAV usually requires adenoviral elements. At the quantity termini of the AAV genome, there are T-shaped hairpin structures called inverted terminal repeats (ITRs). This part of the ITR serves as the origin of replication and functions as a primer. In addition, the ITR is also required for packaging into viral particles and for integration into chromosomal DNA of a host cell. In the left half of the genome, a rep gene encoding non-structural proteins, that is, regulatory proteins involved in replication and transcription (Rep78, Rep68, Rep52, and Rep40) is present, and in the right half of the genome, a cap gene encoding three capsid structural proteins (VP1, VP2, and VP3) is present. The life cycle of AAV is classified into latent infection and lytic infection. The former occurs when AAV infects alone, and is characterized by being incorporated into the AAVS1 region on the long arm of chromosome 19 (19q13.3-qter) of the host cell. This incorporation is due to non-homologous recombination and involves Rep. It has been reported that Rep78/Rep68 binds to a base sequence (GAGC repeat sequence) commonly present in the AAVS1 region and the Rep-binding region of ITR. Accordingly, when wild-type AAV infects a target cell, it is considered that Rep binds to both the ITR of AAV and the AAVS1 region, and that site-specific integration of the AAV genome into chromosome 19 occurs through the involvement of Rep. When a helper virus such as an adenovirus simultaneously infects a cell, or when a helper virus further superinfects a cell in which AAV is latently infected, replication of AAV occurs, and a large amount of virus is released due to cell lysis (lytic infection).

AAV has been reported to have serotypes 1 to 12 based on its capsid. In addition, serotypes such as rh10, DJ, DJ/8, PHP.eB, PHP.S, AAV2-retro, AAV2-QuadYF, AAV2.7m8, AAV6.2, rh.74, AAV2.5, AAV-TT, and Anc80 have also been reported. The AAV-derived construct of the present disclosure may be produced based on an AAV of serotype suitable for the target tissue. For example, the relationship of (serotype): (target tissue) may be selected as follows: (AAV1): (muscle, liver, respiratory tract, neuronal cells); (AAV2):(muscle, liver, neuronal cells); (AAV3) : (muscle, liver, neuronal cells); (AAV4): (muscle, ependymal cells); (AAV5):(muscle, liver, neuronal cells, glial cells, respiratory tract); (AAV6):(muscle, liver, respiratory tract, neuronal cells); (AAV7):(muscle, liver); (AAV8):(muscle, liver); and (AAV9):(muscle, liver, respiratory tract). As the capsid of AAV, in addition to the wild-type, examples thereof include capsids with targeting mutations (such as AAV2i8, AAV2.5, AAV-TT, and AAV9.HR), capsids with random mutations (such as AAV-PHP.B), and capsids designed in silico (such as Anc80), and in one embodiment, the AAV-derived construct of the present disclosure may contain these modified capsids, and the AAV-derived construct plasmid of the present disclosure may be constructed to encode these modified capsids. As used herein, when a nucleic acid sequence is described as being derived from a specific serotype, it is intended that the nucleic acid sequence may encode a wild-type capsid or may encode a modified capsid based on the wild-type capsid as described above.

As used herein, the term "virus-derived" for a nucleic acid means that the nucleic acid has high homology or identity to a nucleic acid present in the virus, and does not require that the nucleic acid be obtained from the virus or prepared using a nucleic acid obtained from the virus. For example, when a nucleic acid or a translation product thereof has a sequence having high identity (for example, at least 75%, at least 80%, at least 85%, at least 90%, at least 91%, at least 92%, at least 93%, at least 94%, at least 95%, at least 96%, at least 97%, at least 98%, or at least 99% identity) to a nucleic acid present in a virus or a translation product thereof, the nucleic acid may be considered to be derived from a virus.

As used herein, the term "Rep" refers to a Rep gene of an adeno-associated virus (AAV), a portion thereof, or a variant thereof. The wild-type AAV Rep encodes regulatory proteins (Rep78, Rep68, Rep52, and Rep40) involved in replication and transcription, and the Rep of the present disclosure can encode a protein having a function equivalent to at least one of Rep78, Rep68, Rep52, and Rep40. AAV has been reported to have serotypes 1 to 12 based on its capsid, and other serotypes such as rh10, DJ, DJ/8, PHP.eB, PHP.S, AAV2-retro, AAV2-QuadYF, AAV2.7m8, AAV6.2, rh.74, AAV2.5, AAV-TT, and Anc80 have also been reported. Rep of the present disclosure can be derived from a Rep of an AAV of any serotype. The Rep of the present disclosure or a gene product thereof (such as a protein) may have a sequence (a base sequence or an amino acid sequence) having high identity (for example, at least 75%, at least 80%, at least 85%, at least 90%, at least 91%, at least 92%, at least 93%, at least 94%, at least 95%, at least 96%, at least 97%, at least 98%, or at least 99% identity) to a Rep of an AAV of any serotype or a gene product thereof (such as a protein).

As used herein, the "repeat sequence" or "tandem repeat" (a nucleic acid sequence) is a generic term for a nucleic acid sequence of an organism genome in which the same sequence is repeatedly (particularly several times or more) found. Any repeat sequence used in the art can be used in the present disclosure. Typically, a promoter sequence appears repeatedly.

As used herein, the term "terminal repeat sequence" is a general term referring to a nucleic acid sequence in a biological genome in which the same sequence is repeated (particularly multiple times) and is present at a terminal. Examples of the terminal repeat sequences include, but are not limited thereto, an inverted terminal repeat (ITR) sequence and a long terminal repeat (LTR) sequence. The terminal repeat sequence may be derived from any of adenoviruses of serotypes 1 to 52, adeno-associated viruses of serotypes 1 to 12, or variants thereof (for example, rh10, DJ, DJ/8, PHP.eB, PHP.S, AAV2-retro, AAV2-QuadYF, AAV2.7m8, AAV6.2, rh.74, AAV2.5, AAV-TT, and Anc80). The terminal repeat sequence may be a naturally occurring sequence or an artificially mutated sequence. A sequence having an "artificial mutation" may be produced, for example, by introducing an appropriate mutation into the sequence of a naturally occurring sequence described in publicly known documents.

As used herein, the term "helper gene" refers to any gene that increases AAV yield in a producer cell. For example, in a case where HEK293 cells (ATCC: CRL-1573) are introduced with pAAV-ZsGreen (TAKARA: 6231) and pRC1 (TAKARA: 6672), and cells with or without the introduction of a candidate gene are prepared, these cells are cultured in DMEM (Thermo Fisher: 11965) (2% FBS (Thermo Fisher: 10270) and 1% penicillin-streptomycin mixed solution (NACALAI TESQUE, INC.: 09367-34)) medium at 37°C in a 5% $CO_2$ incubator, after 3 days, 5 μL of Nuclease-Free Water (Promega: P1193), 1 μL of 10 × React Buffer (with $MgCl_2$) (Thermo Fisher: ED0521), and 2 μL of DNase I (Thermo Fisher: ED0521) are added to 2 μL of the collected supernatant and reacted at 37°C for 30 minutes in a block incubator, then, 90 μL of Nuclease-Free Water (0.001% Pluronic (registered trademark) F-68 (100X) (Thermo Fisher: 24040032) and 5 mM EDTA (TAKARA: T9191)) are added and reacted at 95°C for 10 minutes in a block incubator, to 2 μL of the solution prepared by 100-fold dilution of the obtained solution with Nuclease-Free Water (0.001% Pluronic (registered trademark) F-68 (100X)), 2 μL of Nuclease-Free Water, 5 μL of PowerUp (registered trademark) SYBR (registered trademark) Green Master Mix (Thermo Fisher: A25780), 0.5 μL of 10 μM CMV-F primer (CATCAATGGGCGTGGATAGC: SEQ ID NO: 1), and 0.5 μL of 10 μM CMV-R primer (GGAGTTGTTACGACATTTTGGAAA: SEQ ID NO: 2) are added, after 95°C for 10 minutes, qPCR is performed using QuantStudio 3 Real-Time PCR System Fast96-well (Thermo Fisher: QS3-96F) under the conditions of repetition of 40 cycles of 95°C for 15 seconds, 55°C for 5 seconds, and 72°C for 30 seconds, and the amount of AAV particles is measured, when the amount of AAV particles in cells into which a candidate gene is introduced is increased by at least 50% compared to cells into which the candidate gene is not introduced, the candidate gene can be determined to be a helper gene. Typically, the helper gene is selected from the adenoviral E1A, E1B, E2A, VA, or E4, a portion thereof, or a variant thereof. To date, 52 antigenic types (serotypes) of human adenovirus have been identified. The helper gene of the present disclosure may be derived from a helper gene of an adenovirus of any antigenic type. The helper gene of the present disclosure or a gene product thereof (such as a protein) may have a sequence (a base sequence or an amino acid sequence) having high identity (for example, at least 75%, at least 80%, at least 85%, at least 90%, at least 91%, at least 92%, at least 93%, at least 94%, at least 95%, at least 96%, at least 97%, at least 98%, or at least 99% identity) to a helper gene of an adenovirus of any serotype or a gene product thereof (such as a protein).

As used herein, the term "regulation of Rep gene expression" refers to any operation that increases or decreases the amount of Rep gene product (for example, Rep gene transcript). For example, in the same cell, the same culture medium, or the same isolated space (such as a well or dish), when the amount of Rep gene product or the ratio of the amount of the Rep gene product to the Rep gene at different time points (for example, 6 hours, 12 hours, 24 hours, or 72 hours apart) varies by at least 30%, the expression of the Rep gene is considered to be regulated.

As used herein, the term "regulation of helper gene expression" refers to any operation that increases or decreases the amount of helper gene product (for example, gene transcript). For example, in the same cell, the same culture medium, or the same isolated space (such as a well or dish), when the amount of helper gene product or the ratio of the amount of the helper gene product to the helper gene at different time points (for example, 6 hours, 12 hours, 24 hours, or 72 hours apart) varies by at least 30%, the expression of the helper gene is considered to be regulated. A step of regulating expression of the helper gene may include, for example, providing a stimulus corresponding to a stimulus-responsive promoter operably linked to the helper gene (drug, light, or the like).

As used herein, the term "expression control system" for a gene refers to a substance, a portion of the substance, or a combination of these substances that can, under specific conditions, initiate or terminate transcription and/or translation of the gene, or induce degradation of a nucleic acid containing the gene or a gene product thereof. Examples of the expression control system include a combination of a stimulus (for example, a drug or light) and a promoter responsive to the stimulus (for controlling transcription of a gene), a combination of Cre protein and a Cre-responsive nucleic acid region (LoxP) (for controlling transcription of a gene), a CRISPR-Cas9 system (for degradation of a nucleic acid containing a gene), siRNA or shRNA (for degradation of a gene product), and a ubiquitin ligase (for degradation of a gene product). When the expression control system includes nucleic acid substances such as a promoter and a nucleic acid region having a specific function, it may be referred to as a nucleic acid element of the expression control system. When a cell, a culture, or an isolated space includes a nucleic acid element of an expression control system for a helper gene, the expression of the helper gene is considered to be regulated. Typically, the expression control system can be switched on and/or off by adding, to the target cell, a substance that is not naturally present in the target cell.

As used herein, the phrase "two genes are in cis" refers to a state in which these genes are present on the same nucleic acid molecule or a nucleic acid molecule of a complementary strand (in the case of double-stranded nucleic acid). In addition, as used herein, the phrase "two genes are in trans" refers to a state in which, in a certain cell (in a certain organism), these genes are not present on the same nucleic acid molecule or a nucleic acid molecule of a complementary strand (in the case of double-stranded nucleic acid). For example, a gene present on the genome and a gene on a nucleic acid introduced by a virus-derived construct may be in trans. If necessary, whether two genes are in trans may be determined based on the state at the time when the two genes come to be simultaneously present in a cell (for example, by introduction of a nucleic acid into the cell).

As used herein, when referring to the number of genes, a single gene refers to a gene that has a contiguous sequence in a form that is normally (with the highest frequency or a probability of 50% or more) present on the genome of a certain organism. For example, two exons encoding a certain protein may be two genes. For example, when a promoter sequence and a sequence encoding a protein form a continuous sequence, a nucleic acid segment including the promoter sequence and the sequence encoding the protein may be a single gene. For example, when a protein that becomes functional upon cleavage is encoded by a continuous sequence on the genome, the protein may be encoded by a single gene. When referring to a gene in terms of function, the nucleic acid sequence is not required to be a continuous sequence, and, for example, a plurality of exons encoding a certain protein are collectively referred to as the gene for the protein.

As used herein, the "deficient" of a gene refers to that a nucleic acid does not contain the gene or contains a gene that is modified so as not to perform the normal function (for example, the function of producing a functional protein) of the gene.

As used herein, the "operably linked" refers to placing the expression (actuation) of a desired sequence under the control of a transcriptional and translational regulatory sequence (for example, a promoter, an enhancer, or the like) or a translational regulatory sequence. In order for a promoter to be operably linked to a gene, the promoter is usually located immediately upstream of the gene, but is not necessarily located adjacent to the gene.

As used herein, the "plasmid" refers to a circular DNA that is present separately from chromosomes in a cell or that is present separately from chromosomes when introduced into a cell.

As used herein, the term "cationic substance" refers to a substance that carries a positive charge when added to neutral water, and is typically provided in a complex with a nucleic acid (such as a plasmid).

As used herein, the term "virus-derived construct" refers to a construct that includes a nucleic acid construct having a structure at least partially derived from a virus or a protein produced therefrom. Examples of the virus-derived construct include viral vectors, virus-like particles (VLPs), oncolytic viruses (including those modified to proliferate only in cancer cells), and viral replicons (self-replicating viral genomes lacking the ability to produce viral particles).

As used herein, the term "viral vector" refers to a construct that at least partially has a structure derived from a virus and is capable of introducing a nucleic acid into a target cell. Typically, a viral vector is in the form of a viral particle containing a viral structural protein and a nucleic acid containing a heterologous gene.

As used herein, the term "producer cell" refers to a cell capable of producing a desired virus-derived construct, and may be a cell in which genes necessary for production of the virus-derived construct are expressed from a chromosome and an introduced plasmid. A desired virus-derived construct may be produced by introducing a plasmid into a producer cell. For example, in the case of an AAV viral vector, E1A and E1B are required for its production, but since HEK293 cells have these, they can be omitted from the helper plasmid. Other cells also function as producer cells when modified to have such functional auxiliary factors.

As used herein, the term "animal cell" refers to a cell obtained from an animal (such as a human, mouse, rat, monkey, or dog), or a cell modified from a cell obtained from an animal.

As used herein, the "host cell" refers to a cell into which an exogenous nucleic acid, protein, virus, or virus-derived construct is introduced (including the progeny of such a cell).

As used herein, the "protein", "polypeptide", and "peptide" are used in the same meaning, and refer to an amino acid polymer of any length. The polymer may be linear, branched, or cyclic. An amino acid may be a natural, non-natural, or modified amino acid. The term also encompasses a natural or artificially modified polymer. Examples of such a modification include disulfide bond formation, glycosylation, lipidation, acetylation, phosphorylation, and any other manipulation or modification (for example, conjugation with a labeling component).

As used herein, the term "transcriptional and translational regulatory sequence" collectively refers to promoter sequences, polyadenylation signals, transcription termination sequences, upstream regulatory domains, origins of replication, enhancers, IRES, and the like, which cooperate to enable replication, transcription, and translation of a coding sequence in a recipient cell. Not all of the transcriptional and translational regulatory sequences are necessarily required, as long as replication, transcription, and translation of the selected coding sequence are possible in a suitable host cell. Those skilled in the art can readily identify a regulatory nucleic acid sequence from publicly available information. Furthermore, those skilled in the art can identify a transcriptional and translational regulatory sequence applicable to the intended use, for example, in vivo, ex vivo, or in vitro.

As used herein, the term "promoter" refers to a segment of a nucleic acid sequence that controls transcription of an operably linked nucleic acid sequence. The promoter includes a specific sequence sufficient for recognition, binding, and initiation of transcription by RNA polymerase. The promoter may have a sequence that regulates recognition, binding, or initiation of transcription by RNA polymerase.

As used herein, the term "enhancer" refers to a segment of a nucleic acid sequence that functions to enhance the expression efficiency of a target gene.

As used herein, the term "silencer" refers to a segment of a nucleic acid sequence that functions, in contrast to an enhancer, to reduce the expression efficiency of a target gene.

As used herein, the term "insulator" refers to a segment of a nucleic acid sequence that has a cis-regulatory function of regulating the expression of genes located at distant positions on the DNA sequence.

As used herein, the term "terminator" refers to a segment of a nucleic acid sequence that is located downstream of a protein-coding region and is involved in the termination of transcription when the nucleic acid is transcribed into mRNA.

As used herein, the "origin of replication" refers to a segment of a nucleic acid sequence to which a protein (for example, initiator DnaA protein or the like) that recognizes the nucleic acid sequence binds or to which RNA is synthesized to partially unwind a DNA double helix, and from which replication is initiated.

As used herein, an internal ribosome entry site ("IRES") refers to a nucleic acid segment that promotes the entry or retention of a ribosome during translation of a downstream nucleic acid sequence.

As used herein, the "homology" of nucleic acids refers to a degree of identity of two or more nucleic acid sequences with respect to one another, and having "homology" generally refers to having a high degree of identity or similarity. Therefore, the identity or similarity of the sequences is higher as homology of two nucleic acids is high. The "similarity" is a numerical value calculated for a similar base in addition to identity, and here, the similar bases refer to a case where some bases in a mixed base (for example, R=A+G, M=A+C, W=A+T, S=C+G, Y=C+T, K=G+T, H=A+T+C, B=G+T+C, D=G+A+T, V=A+C+G, and N=A+C+G+T) are identical. Whether two types of nucleic acids have homology can be determined by a direct comparison of sequences or a hybridization method under stringent conditions. When two nucleic acid sequences are directly compared, the genes have homology when the genes are typically at least 50% identical, preferably at least 70% identical, and more preferably, at least 80%, 90%, 95%, 96%, 97%, 98%, or 99% identical between the nucleic acid sequences.

Amino acids may be mentioned herein by either their commonly known three letter symbols or their one character symbols recommended by the IUPAC-IUB Biochemical Nomenclature Commission. Similarly, nucleotides may be mentioned by their commonly recognized one character codes. As used herein, a comparison of similarity, identity, and homology of an amino acid sequence and a base sequence is calculated by using a sequence analysis tool BLAST and default parameters. For example, identity can be searched using BLAST 2.10.1+ (published on June 18, 2020) of the NCBI. As used herein, a value for identity generally refers to a value obtained when aligned under the default conditions using BLAST described above. However, when a higher value is obtained by changing a parameter, the highest value is set as the value of identity. In a case where identity is evaluated in a plurality of regions, the highest value in the plurality of regions is set as the value of identity. Similarity is a numerical value calculated by taking into consideration a similar amino acid in addition to identity.

As used herein, unless otherwise specified, it is understood that reference to a biological material (for example, a protein, a nucleic acid, or a gene) is also a reference to a variant of the biological material (for example, a variant having a modification in a base sequence) that performs a function similar to (but not to the same extent as) the biological function of the biological material. Such a variant can contain a fragment of the original molecule, and a molecule that is at least 30%, 40%, 50%, 60%, 70%, 80%, 90%, 95%, 98%, or 99% identical as compared to the sequence of the original molecule that is aligned over the amino acid sequence or nucleic acid sequence of the original biological material of the same size, or aligned by computer homology programs known in the art. The variant may include molecules having modified nucleotides (for example, modification by methylation).

As used herein, the "corresponding" amino acid or nucleic acid refers to an amino acid or a nucleotide which has or is expected to have, in a certain polypeptide molecule or polynucleotide molecule, a similar action as a predetermined amino acid or nucleotide in a reference polypeptide or a polynucleotide for comparison, and, particularly in the case of enzyme molecules, refers to an amino acid which is present at a similar position in an active site and makes a similar contribution to catalytic activity. For example, for an antisense molecule, it can be a similar moiety in an ortholog corresponding to a specific moiety of the antisense molecule. A corresponding amino acid can be a specific amino acid subjected to, for example, cysteination, glutathionylation, S-S bond formation, oxidation (for example, oxidation of a methionine side chain), formylation, acetylation, phosphorylation, glycosylation, myristylation, or the like. Alternatively, a corresponding amino acid can be an amino acid responsible for dimerization. Such a "corresponding" amino acid or nucleic acid may be a region or a domain over a certain range. Accordingly, it is referred herein as a "corresponding" region or domain in such a case.

As used herein, the "corresponding" gene (for example, a polynucleotide sequence or molecule) refers to a gene (for example, a polynucleotide sequence or molecule) in a certain species which has or is expected to have a similar action as a predetermined gene in a reference species for comparison. When a plurality of genes having such an action are present, the corresponding gene refers to a gene having the same evolutionary origin. Accordingly, a gene corresponding to a certain gene may be an ortholog of such a gene. For example, the cap of AAV serotype 1 may correspond to the cap of AAV serotype 2. For example, a corresponding gene in a certain virus can be found by search on a sequence database for the virus using the gene sequence of a reference virus of the corresponding gene as a query sequence.

In accordance with the present disclosure, as used herein, the term "activity" refers to a function of a molecule in its broadest sense. Activity, although not intended to be limiting, generally includes a biological function, biochemical function, physical function, and chemical function of a molecule. Examples of the activity include enzymatic activity, an ability to interact with another molecule, an ability to activate, promote, stabilize, inhibit, suppress, or destabilize a function of another molecule, stability, and an ability to localize at a specific position in a cell. When applicable, the term also relates to a function of a protein complex in the broadest sense.

As used herein, when referring to a certain gene or a nucleic acid molecule or a polypeptide related thereto, the "biological function" refers to a specific function that the gene, nucleic acid molecule, or polypeptide can have in vivo, and examples thereof include, but are not limited to, a specific cell surface structure recognition ability, an enzyme activity, and a binding ability to a specific protein. In the present disclosure, for example, a function in which a certain promoter is recognized in a specific host cell can be exemplified, but the present disclosure is not limited thereto. As used herein, the biological function can be exerted by "biological activity". As used herein, the "biological activity" refers to an activity that a certain agent (for example, a polynucleotide, a protein, or the like) can have in a living body. Biological activity encompasses an activity of exerting a variety of functions (for example, transcription promoting activity), and also encompasses, for example, an activity of activating or inactivating another molecule by an interaction with a certain molecule. For example, when a certain factor is an enzyme, the biological activity thereof encompasses enzyme activity thereof. In another example, in a case where a certain factor is a ligand, binding to a receptor corresponding to the ligand is encompassed. Such biological activity can be measured by a technique that is well known in the art.

As used in the present disclosure, the term "infectivity" of a virus or a virus-derived construct refers to the ability of the virus or the virus-derived construct to introduce nucleic acid contained therein into a cell through attachment to the cell or membrane fusion of the virus or the virus-derived construct. The term "replicative ability" of a virus or a virus-derived construct refers to the ability to produce infectious virus particles or virus-derived construct particles in an infected cell.

As used herein, the terms "transformation", "transduction", and "transfection" are used interchangeably, unless otherwise stated, and refer to the introduction of a nucleic acid into a host cell (optionally via a virus or a virus-derived construct). As a transformation method, any method can be used as long as it is a method of introducing a nucleic acid into a host cell, and examples thereof include various well-known techniques such as use of competent cells, an electroporation method, a method using a particle gun (gene gun), and a calcium phosphate method.

As used herein, the "purified" substance or biological factor (for example, a nucleic acid, a protein, or the like) refers to a substance or a biological factor in which at least a part of a factor naturally accompanying the biological factor is removed. Thus, the purity of the biological factor in the purified biological factor is generally higher than the purity in the normal state of the biological factor (that is, concentrated). The "purified" as used herein refers to the presence of preferably at least 75% by weight, more preferably at least 85% by weight, still more preferably at least 95% by weight, and most preferably at least 98% by weight of a biological factor of the same type. The substance used in the present disclosure is preferably a "purified" substance.

As used herein, the term "pharmaceutical ingredient" refers to any component that can constitute a pharmaceutical composition, and examples thereof include active ingredients (which themselves exhibit a pharmacological effect), and additive ingredients (which themselves are not expected to exhibit a pharmacological effect but are expected to play certain roles (for example, excipients, lubricants, surfactants, and the like) when contained as a medicine). The pharmaceutical ingredient may be a single substance or a combination of a plurality of substances and agents. Any combination, such as a combination of an active ingredient and an additive ingredient, or a combination of an adjuvant and an active ingredient, may also be included.

As used herein, the term "active ingredient" refers to an ingredient that exerts an intended pharmacological effect, and may refer to a single component or a plurality of components.

As used herein, the term "additive ingredient" refers to any component that is not expected to exert a pharmacological effect but plays a certain role when contained as a medicine, and examples thereof include pharmaceutically acceptable carriers, stabilizers, (co)adjuvants, solubility enhancers, solubilizers, diluents, excipients, buffers, binders, diluents, flavoring agents, and lubricants.

As used herein, a "drug", "agent", or "factor" (all the terms correspond to an "agent" in English) is used interchangeably in a broad sense, and may be any substance or other elements (for example, energy such as light, radioactivity, heat, and electricity) as long as the intended object can be achieved. Examples of such a substance include, but are not limited to, a protein, a polypeptide, an oligopeptide, a peptide, a polynucleotide, an oligonucleotide, a nucleotide, a nucleic acid (including, for example, DNA such as cDNA or genomic DNA, RNA such as mRNA), a polysaccharide, an oligosaccharide, a lipid, an organic small molecule (for example, a hormone, a ligand, an information transmitting substance, an organic small molecule, a molecule synthesized by combinatorial chemistry, a small molecule that can be used as a medicine (for example, a small molecule ligand or the like), or the like), a composite molecule thereof, and a mixture thereof.

As used herein, the "complex" or "complex molecule" means any construct containing two or more moieties. For example, when one moiety is a polypeptide, the other moiety may be a polypeptide or another substance (for example, a substrate, a sugar, a lipid, a nucleic acid, another hydrocarbon, or the like). As used herein, two or more moieties constituting the complex may be bonded by a covalent bond or may be bonded by another bond (for example, a hydrogen bond, an ionic bond, a hydrophobic interaction, van der Waals force, or the like).

As used herein, the term "label" refers to the presence (for example, a substance, energy, an electromagnetic wave, and the like) for identifying a molecule or substance to be targeted from others. Examples of such a labeling method include a radioisotope (RI) method, a fluorescence method, a biotin method, and a chemiluminescence method. When a plurality of target proteins or factors or means for capturing the same are labeled by a fluorescence method, the markers are labeled with fluorescent substances having different maximum fluorescence emission wavelengths. A difference in maximum fluorescence emission wavelength is preferably 10 nm or more. Any label that does not affect the function can be used, and examples of the fluorescent substance include Alexa™ Fluor. Alexa™ Fluor is a water-soluble fluorescent dye obtained by modifying coumarin, rhodamine, fluorescein, cyanine, or the like, is a series corresponding to a wide range of fluorescence wavelengths, and is significantly stable, bright, and less pH sensitive as compared with other fluorescent dyes having corresponding wavelengths. Examples of a combination of fluorescent dyes having a maximum fluorescence wavelength of 10 nm or more include a combination of Alexa™ 555 and Alexa™ 633 and a combination of Alexa™ 488 and Alexa™ 555. Examples of other fluorescent labels include cyanine dyes (for example, Cy3 and Cy5 of CyDye™ series, and the like), rhodamine 6G reagents, N-acetoxy-N2-acetylaminofluorene (AAF), and AAIF (an iodine derivative of AAF). In the present disclosure, such a label can be utilized to modify the object to be targeted so that the object can be detected by detection means used. Such modifications are known in the art and those skilled in the art can carry out such methods as appropriate for the label and depending on the object to be targeted.

As used herein, the "kit" refers to a unit generally providing portions to be provided (for example, a virus-derived construct, instructions, and the like) that are usually divided into two or more sections. The form of the kit is preferred when it is intended to provide a composition that should not be provided in a mixed state for stability or the like, but is preferably mixed immediately before use. Such a kit preferably includes an instruction or manual describing how to use the provided portions or how a reagent should be processed. When the kit is used herein as a reagent kit, the kit generally includes an instruction or the like describing how to use a plasmid and the like.

As used herein, the "instruction" is a document with an explanation of the method of use of the present disclosure for a physician or other users. The instruction includes wording that instructs administration of the medicine or the like of the present disclosure. In addition, the instruction may include wording that instructs the administration form. The instruction is prepared in accordance with a format defined by the regulatory agency of the country in which the present disclosure is practiced (for example, the Ministry of Health, Labor and Welfare in Japan, Food and Drug Administration (FDA) in the United States or the like), with an explicit description showing approval by the regulatory agency. The instruction is a so-called package insert and is usually provided in, but is not limited to, a paper medium. For example, the instruction may also be provided in a form such as an electronic medium (for example, a web site or an e-mail provided on the Internet).

As used herein, the "change in (Gibbs) free energy (ΔG)" is thermodynamically or statistically described by the following Equations (1) and (2).

$$\Delta G = \Delta H - T\Delta S \quad (1)$$

$$\Delta G = \Delta G^\circ + RT\ln K \quad (2)$$

Here, K is an equilibrium constant, and ΔG° is the change in Gibbs free energy in a standard state (1 atm, 25°C). When the equilibrium constant of the nucleic acid is considered, it can be described as an equilibrium reaction in which two strands A and B (for example, 3' UTR and 5' UTR) associate at a ratio of 1:1 (Equation (3)).

$$A + B \leftrightarrow B \quad (3)$$

When the state molar fraction of the double strand is α and the total concentration of the nucleic acid is C, the concentrations [A] and [B] of A and B when completely dissociated are C/2, and thus, the equilibrium constant can be represented by the following Equation (4).

$$K = 2\alpha/((1 - \alpha)^2 \times C) \quad (4)$$

When the nucleic acid is in an equilibrium state deviated by 50%,

$$\alpha = 1/2,\ \Delta G = 0 \quad (5);$$

thus, by substituting Equations (4) and (5) into Equation (2), the following Equation (6) is obtained.

$$\Delta G^\circ = -RTm\ln\ (4/C) \quad (6)$$

By substituting Equation (6) into Equation (1), the following Equation (7) is obtained.

$$1/Tm = (R/\Delta H^\circ)\ln(Ct/4) + \Delta S^\circ/\Delta H^\circ \quad (7)$$

When the measured values are plotted as two functions of 1/Tm and ln(C/4), the change in enthalpy ΔH° and the change in entropy ΔS° in the standard state are obtained from the intercept and the slope.

The term "about" refers to the indicated value plus or minus 10%. The "about" when used for a temperature refers to an indicated temperature plus or minus 5°C, and the "about" when used for a pH refers to an indicated pH plus or minus 0.5.

(Preferred embodiments)

Preferred embodiments of the present disclosure will be described below. It is understood that the embodiments provided below are provided for a better understanding of the present disclosure, and that the scope of the present disclosure should not be limited to the following description. Therefore, it is apparent that those skilled in the art can appropriately make modifications within the scope of the present disclosure in view of the description herein. In addition, it is also understood that the following embodiments may be used alone or in combination.

In one aspect, the present disclosure provides a nucleic acid containing a Rep gene derived from an adeno-associated virus (AAV) of a first serotype, and an inverted terminal repeat (ITR) sequence derived from an AAV of a second serotype different from the first serotype and having a ΔG value in a range of -65 to -95 kcal/mol. The interaction between the ITR and Rep is involved in genome replication and genome packaging of AAV (Chiorini J. A., et al., JOURNAL OF VIROLOGY (1999)). Therefore, when the ITR and Rep do not interact with each other, an AAV is not produced and an AAV genome is not packaged. Therefore, the present inventors focused on the change in free energy (ΔG) of the ITR and found that an ITR having a free energy change within a specific range enhances the AAV vector productivity. Accordingly, the nucleic acid of the present disclosure exhibits high AAV vector productivity. Any means for achieving this is intended to fall within the scope of the present disclosure. For example, even in the absence of an explicit description, a description of a method using a certain component is also intended to encompass embodiments that reflect other means, such as a composition containing the component, the use of the component, or the component for use in the method.

In some embodiments, the Rep gene may be derived from an AAV of serotype 2 (hereinafter, also referred to as Rep2, Rep-2, or the like). A nucleic acid containing a component derived from an AAV of serotype 2 and an ITR having a specific change in free energy (ΔG) exhibits high AAV vector productivity. In some embodiments, the value of the change in free energy (ΔG) of the ITR may be -65 to -95 kcal/mol, preferably -75 to -95 kcal/mol, more preferably -80 to -90 kcal/mol, and most preferably -80 to -88 kcal/mol. In certain embodiments, the Rep gene may be derived from an AAV of serotype 2, and the ITR may be derived from an AAV of any one of serotypes 1 to 7, and preferably derived from an AAV of serotype 1, 6, or 7 (hereinafter, may also be referred to as ITR1, ITR-1, or the like).

In some embodiments, the change in free energy (ΔG (kcal/mol)) can be calculated by a nucleic acid secondary structure prediction program, for example, mfold (GCG Software), IPknot, CentroidFold (https://www.ncrna.org/), ViennaRNA (http://rna.tbi.univie.ac.at/), RNALOSS (P Clote, RNALOSS: a web server for RNA locally optimal secondary structures, Nucleic Acids Res. 2005 Jul 1; 33: W600-4.), or RNA Secondary structure prediction (http://www.genebee.msu.su/services/rna2_reduced.html).

In some embodiments, the ITR sequence may be derived from an AAV of any one of serotypes 1 to 7. In a preferred embodiment, the ITR sequence may be derived from an AAV of any one of serotypes 1 to 4, 6, and 7.

In some embodiments, the Rep gene may be derived from an AAV of any one of serotypes 1 to 12. In some embodiments, the Rep gene may be derived from an AAV of serotype 1, 2, 3, 5, 7, 8, or 10. In a preferred embodiment, the Rep gene may be derived from an AAV of serotype 1, 2, 5, 7, or 10. Since Rep4 has high homology with Rep3, Rep6 has high homology with Rep1, and Rep9 has high homology with Rep7, it is expected that the use of each of Rep3, Rep1, and Rep7 can achieve equivalent AAV production. Accordingly, in one embodiment, the present disclosure may include a Rep gene selected from the group consisting of Rep1/6, Rep2, Rep3/4, Rep12, Rep5, Rep8, Rep7/9, and Rep10/11. Note that the notation "Rep1/6," "Rep3/4," and "Rep7/9" is used because they are similar to each other (having 90% identity at the amino acid sequence level) and may be classified into the same family depending on the classification method. In addition, in another embodiment, the Rep gene may encode an amino acid sequence having 90% or more identity to at least one of Rep3, Rep1, or Rep7.

In a specific embodiment, the Rep gene is derived from an AAV of serotype 1, and the ITR sequence may be derived from an AAV of any one of serotypes 1 to 7.

In a specific embodiment, the Rep gene is derived from an AAV of serotype 2, and the ITR sequence may be derived from an AAV of any one of serotypes 1 to 7.

In a specific embodiment, the Rep gene is derived from an AAV of serotype 3, and the ITR sequence may be derived from an AAV of any one of serotypes 1 to 7.

In a specific embodiment, the Rep gene is derived from an AAV of serotype 4, and the ITR sequence may be derived from an AAV of any one of serotypes 1 to 7.

In a specific embodiment, the Rep gene is derived from an AAV of serotype 5, and the ITR sequence may be derived from an AAV of any one of serotypes 1 to 7.

In a specific embodiment, the Rep gene is derived from an AAV of serotype 6, and the ITR sequence may be derived from an AAV of any one of serotypes 1 to 7.

In a specific embodiment, the Rep gene is derived from an AAV of serotype 7, and the ITR sequence may be derived from an AAV of any one of serotypes 1 to 7.

In a specific embodiment, the Rep gene is derived from an AAV of serotype 8, and the ITR sequence may be derived from an AAV of any one of serotypes 1 to 7.

In a specific embodiment, the Rep gene is derived from an AAV of serotype 9, and the ITR sequence may be derived from an AAV of any one of serotypes 1 to 7.

In a specific embodiment, the Rep gene is derived from an AAV of serotype 10, and the ITR sequence may be derived from an AAV of any one of serotypes 1 to 7.

In some embodiments, the nucleic acid of the present disclosure may further contain a Cap gene (for example, a Cap gene derived from an AAV of any one of serotypes 1 to 10). In a preferred embodiment, the Cap gene may be derived from an AAV of serotype 6 or 9.

A preferred combination of the Rep gene and the ITR sequence may be Rep1/ITR1, Rep1/ITR2, Rep1/ITR3, Rep1/ITR4, Rep1/ITR6, Rep1/ITR7, Rep2/ITR1, Rep2/ITR2, Rep2/ITR3, Rep2/ITR4, Rep2/ITR5, Rep2/ITR6, Rep2/ITR7, Rep5/ITR5, Rep7/ITR1, Rep7/ITR2, Rep7/ITR3, Rep7/ITR4, Rep7/ITR6, Rep7/ITR7, Rep10/ITR1, Rep10/ITR2, Rep10/ITR3, Rep10/ITR4, Rep10/ITR6, or Rep10/ITR7.

In a further embodiment, the combination of the Rep gene and the ITR sequence may include Rep6/ITR1, Rep6/ITR2, Rep6/ITR3, Rep6/ITR4, Rep6/ITR6, Rep6/ITR7, Rep9/ITR1, Rep9/ITR2, Rep9/ITR3, Rep9/ITR4, Rep9/ITR6, and Rep9/ITR7.

In some embodiments, the Rep and the ITR may be contained in separate nucleic acids. In some embodiments, the nucleic acid contains a 3' ITR and a 5' ITR, which may be derived from the same AAV serotype or from different AAV serotypes.

The AAV vector productivity by the nucleic acid of the present disclosure may be measured by qPCR targeting a CMV promoter contained in the nucleic acid of the present disclosure, may be measured as an empty ratio and/or a full ratio by quantitative analysis using HPLC, or may be measured as an empty ratio and/or a full ratio by a mass photometry method using Refeyn One (Life Science Solutions). In HPLC, Retention time (min) is shown on the x-axis and Counts is shown on the y-axis, and the empty ratio and the full ratio were calculated from the relative area ratio. In Refeyn, Mass (kDa) is shown on the x-axis and Counts on the y-axis. The range from 3,000 to 4,000 kDa was defined as Empty, and the range from 4,010 to 5,000 kDa was defined as Full. The ratios of the number of vectors were defined as an empty ratio and a full ratio, respectively.

In some embodiments, the nucleic acid of the present disclosure may further contain a helper gene derived from an adenovirus (AdV). In one embodiment, the helper gene includes a gene selected from E1B, E2, E4, and VA of an adenovirus. In one embodiment, the helper gene includes an E1B gene of an adenovirus. In one embodiment, the helper gene includes two or more helper genes related to the production of an adeno-associated virus (AAV)-derived construct, and, if necessary, the helper gene may be selected from E1A, E1B, E2, E4, and VA. In one embodiment, the helper gene includes a helper gene selected from E1A and E1B, and a helper gene selected from E2, E4, and VA.

In some embodiments, the same nucleic acid molecule or nucleic acid duplex may contain the Rep gene, the ITR sequence, and a Cap gene derived from an AAV. In a further embodiment, the same nucleic acid molecule or nucleic acid duplex may contain a Rep gene, an ITR sequence, a Cap gene derived from an AAV, a helper gene derived from an adenovirus (AdV), or any combination thereof.

In some embodiments, a combination of the Rep gene and the ITR sequence is a combination of the Rep gene and the ITR sequence, which has an ability to produce a viral vector with a titer [GC/mL] that is twice or more than that of a combination of the said Rep gene and ITR-5, when measured by qPCR targeting a desired protein such as a CMV promoter or EGFP. For example, when Rep1 is selected as the Rep gene, a preferred combination of Rep1 and a specific ITR sequence may have an ability to produce a viral vector with a titer that is twice or more than that of the combination of Rep1 and ITR-5.

In some embodiments, the nucleic acid of the present disclosure may be a plasmid.

In some embodiments, the nucleic acid of the present disclosure may form a complex with a cationic substance such as a polymer or a liposome. Examples of the cationic substance include, but are not limited to, PEI pro, FectoVIR-AAV, Lipofectamine 2000, Lipofectamine 3000, FuGENE6, FuGENE HD, and FuGENE 4K.

In a further aspect, the present disclosure provides a composition for producing a viral vector, the composition containing the nucleic acid.

In a further aspect, the present disclosure provides a method for synthesizing a nucleic acid, the method including a step of combining a nucleic acid containing a Rep gene and a nucleic acid containing an ITR.

In a further aspect, the present disclosure provides a method for producing a viral vector, the method including: a step of introducing the nucleic acid or the composition into a producer cell; and a step of forming a viral vector in the producer cell. At least a part of the nucleic acid may be integrated into a chromosome of the producer cell. In a further aspect, the present disclosure provides a producer cell or a viral vector produced by the method.

In one embodiment, the present disclosure provides a producer cell containing a nucleic acid containing a Rep gene and a nucleic acid containing an ITR. Examples of the producer cell include, but are not limited to, human embryonic kidney cells (produced by transfecting human fetal kidney cells with DNA cleaved from adenovirus type 5), 911 cells, PER.C6 cells, E1-transformed amniotic cells, E1-transformed A549 cells, GH329: HeLa cells, HEK293 cells, IT293SF cells, HEK293T, HEK293F, Vero cells, CHO cells, Sf9 cells, FreeStyle (trademark) 293-F, Expi293-F (trademark), Expi293 inducible, Expi293 NGT-Viral Production Cells 1.0, Viral Production Cells 2.0 (VPC2.0 cells), AAVpro (registered trademark) 293T Cell Line, Lenti-X (trademark) 293T Cell Line, FreeStyle (trademark) CHO-S cells, ExpiCHO-S (trademark), VirusExpress (trademark) 293 AAV Production Cells, and VirusExpress (trademark) 293T Lentiviral Production Cells. Examples of the producer cell for producing an AAV-derived construct include, in particular, cells such as HEK293, HEK293T, HEK293F, Hela, and Sf9.

In one embodiment, the cell contains a Rep gene and an ITR sequence in a chromosome, and optionally, may further contain one or more of a helper gene and a nucleic acid element of a gene expression control system for the helper gene in the chromosome. In one embodiment, the cell contains a gene of interest (GOI) and a Cap gene (optionally in the chromosome). In one embodiment, the cell contains a helper gene, an ITR, a GOI, a Cap gene, and a Rep gene (optionally in the chromosome). In one embodiment, the cell contains a helper gene, an ITR, a GOI, a Cap gene, and a Rep gene at one genetic locus on the chromosome.

The nucleic acid of the present disclosure, an element of an expression control system, a cell, a virus-derived construct, or a composition containing any of these can be provided as a kit. In one embodiment, the present disclosure provides a kit for regulating expression of a Rep gene, the kit containing a nucleic acid containing a Rep gene, a nucleic acid containing a helper gene, and an expression control system (particularly its nucleic acid element) for the helper gene. In one embodiment, the present disclosure provides a drug pack or kit including one or more containers filled with one or more components that may be added to the composition of the present disclosure. In some cases, information indicating approval of manufacture, use, or sale for administration to humans by a government agency regulating the manufacture, use, or sale of medicines or biological products in a stipulated form can be appended to such a container.

The formulation procedure for the composition the present disclosure as a medicine or the like is known in the art, and is described, for example, in the Japanese Pharmacopoeia, the United States Pharmacopeia, pharmacopeia of other countries, or the like. Accordingly, those skilled in the art can determine the embodiment, such as the amount to be used, without undue experimentation from the descriptions herein.

As used herein, "or" is used when "at least one or more" of the items listed in the text can be employed. The same applies to "or". When explicitly described herein as "within the range" of "two values", the range also includes the two values themselves.

Reference literatures such as scientific literatures, patents, and patent applications cited herein are incorporated herein by reference to the same extent that the entirety of each document is specifically described.

The present disclosure has been described above with reference to preferred embodiments for easy understanding. Hereinafter, the present disclosure will be described based on examples, but the above description and the following examples are provided for the purpose of illustration only and not for the purpose of limiting the present disclosure. Accordingly, the scope of the present disclosure is not limited to the embodiments or examples specifically described as used herein, but is limited only by the claims.

Examples

For reagents, the specific products described in the examples were used. However, the reagent can be substituted with an equivalent product from another manufacturer (Sigma-Aldrich, Wako Pure Chemical, NACALAI TESQUE, INC., R&D Systems, USCN Life Science INC, or the like).

(Construction of plasmid DNA)

1. SYNp34_pHelper

The pUC19 vector plasmid (Addgene: #500005) was treated with the restriction enzymes (AatII and PciI). The following three fragments (VA, E4, and E2A fragments) were inserted therein using In-Fusion. The VA fragment was prepared using the genome of Ad5 (see ATCC VR-1516) as a template and the following primers: primer sequences 5'-ttagtaagcttccctcctgacgcggtaggaggagggggagggtgccctgcatg-3' (SEQ ID NO: 3) and 5'-ccttttgctcacatgtcaattggtagatgtacct ggacatccaggtgatgccggcg-3' (SEQ ID NO: 4). The E4 fragment was prepared using the genome of Ad5 as a template and the following primers: primer sequences 5'-tgtacaGGCGCGCCgaattcgttttagggcggagtaacttgtatgtgttgggaattgtag-3' (SEQ ID NO: 5) and 5'-agggaagcttactaaacggtacacaggaaacaggagacacaactccaagtg-3' (SEQ ID NO: 6). The E2A fragment was prepared using the genome of Ad5 as a template and the following primers: primer sequences 5'-gaaaagtgccacctgac gtcgcggccgcGCGATCGCggtacccaactccatgctcaacagt ccccaggtacagccce-3' (SEQ ID NO: 7) and 5'-gaattcGGCGCGC Ctgtacagcccgggcgaccgcaccctgtgacgaaagccgcccgcaag-3' (SEQ ID NO: 8).

2. pAAV-ITR1-EGFP

The pUC19 vector plasmid was treated with the restriction enzymes (AatII and PciI). The following two fragments were inserted therein using In-Fusion. Primer sequences 5'-cgaaaagtgccacctAAATAATGATTTTATTTTGACTGATAGT GACCTGTTCGTTGCAACA AATTGATGAGCAA-3' (SEQ ID NO: 9) and 5'-caggccCACCTGCtcagattaCAACTTTTGT ATACAAAGTTGGCATTATAAAAAAGCATTG CTCATCAATTTG-3' (SEQ ID NO: 10) were used, with no template (in the case of the absence of a template, a fragment is formed by annealing and extension between the primers themselves, and since the fragment is short, it is prepared in this method. Primer sequence 5'-gaGCAGGTGggcctgattggccCAACTTTT CTATACAAAGTTGGCATTATAAGAAAGCATT GCTTATCAATTTG-3' (SEQ ID NO: 11), 5'-tggccttttgctcgcAAATAATGA TTTTATTTTGACTGATAGTGACCTGTTCGTTGCAACA AATTGATAAGCAA-3' (SEQ ID NO: 12), no template. The obtained plasmid (pUC19_PaqCI-SfiI) was treated with the restriction enzymes (PaqCI and SfiI). Three fragments were excised by PaqCI, PaqCI, PaqCI, and SfiI, respectively, from a plasmid having the 5' ITR, EGFP, and 3' ITR gene portions of the seed plasmid #2 shown in Table 1 below, and inserted therein by ligation.

[Table 1]

| Seed Plasmid No. | 5'ITR recombinant unit | 3'ITR recombinant unit | Helper recombinant unit | Rep recombinant unit |
|---|---|---|---|---|
| #1 | Modified AAV-2 | AAV-5 | Ad-2 | Modified AAV-2 |
| #2 | AAV-1 | AAV-1 | Ad-12 | AAV-1 |

(continued)

| Seed Plasmid No. | 5'ITR recombinant unit | 3'ITR recombinant unit | Helper recombinant unit | Rep recombinant unit |
|---|---|---|---|---|
| #3 | AAV-2 | AAV-2 | Ad-14 | AAV-2 |
| #4 | AAV-3 | AAV-3 | Ad-5 | AAV-3 |
| #5 | AAV-4 | AAV-4 | Ad-8 | AAV-8 |
| #6 | AAV-5 | Modified AAV-2 | Ad-4 | AAV-5 |
| #7 | AAV-6 | AAV-6 | Ad-40 | AAV-10 |
| #8 | AAV-7 | AAV-7 | Ad-52 | AAV-7 |

3. pAAV-ITR2wt-EGFP

pUC19_PaqCI-SfiI was treated with the restriction enzymes (PaqCI and SfiI). Three fragments were excised by PaqCI, PaqCI, PaqCI, and SfiI, respectively, from a plasmid having the 5' ITR, EGFP, and 3' ITR gene portions of the seed plasmid #3 shown in Table 1, and inserted therein by ligation.

4. pAAV-ITR4-EGFP

pUC19_PaqCI-SfiI was treated with the restriction enzymes (PaqCI and SfiI). Three fragments were excised by PaqCI, PaqCI, PaqCI, and SfiI, respectively, from a plasmid having the 5' ITR, EGFP, and 3' ITR gene portions of the seed plasmid #5 shown in Table 1, and inserted therein by ligation.

5. pAAV-ITR5-EGFP

pUC19_PaqCI-SfiI was treated with the restriction enzymes (PaqCI and SfiI). Three fragments excised by PaqCI, PaqCI, and PaqCI/SfiI, respectively, from a plasmid having the 5' ITR and EGFP of the seed plasmid #6 and the 3' ITR gene region of the seed plasmid #1 shown in Table 1, were inserted therein by ligation.

6. pAAV-ITR6-EGFP

pUC19_PaqCI-SfiI was treated with the restriction enzymes (PaqCI and SfiI). Three fragments were excised by PaqCI, PaqCI, PaqCI, and SfiI, respectively, from a plasmid having the 5' ITR, EGFP, and 3' ITR gene portions of the seed plasmid #7 shown in Table 1, and inserted therein by ligation.

7. pAAV-ITR7-EGFP

pUC19_PaqCI-SfiI was treated with the restriction enzymes (PaqCI and SfiI). Three fragments were excised by PaqCI, PaqCI, PaqCI, and SfiI, respectively, from a plasmid having the 5' ITR, EGFP, and 3' ITR gene portions of the seed plasmid #8 shown in Table 1, and inserted therein by ligation.

8. pAAV-ITR2 mutant-EGFP

pAAV-CMV (Takara 6230) was treated with the restriction enzymes (EagI and BamHI). The following fragments were inserted therein using In-Fusion. Primer sequences 5'-gccCGAATCCCGGCCGGCCACCATGGTGAGCAAG-3' (SEQ ID NO: 11) and 5'-TGCCACCCGTggatccTTACTTGTACAGCTCGTC-3' (SEQ ID NO: 12) were used with the EGFP gene sequence (VectorBuilder) as a template.

9. pR1C6

The following 10.pR2C6 (plasmid #10) was treated with the restriction enzymes (SwaI and NotI). The following fragments were inserted therein using In-Fusion. Primer sequences 5'-ATAAAGTTGgcggccgctcgcgaagcgcctcccac-3' (SEQ ID NO: 13) and 5'-AGCCATacctgatttaaatcatTTATTGCTCAGAA-3' (SEQ ID NO: 14) were used with an artificially synthesized AAV1 Rep gene as a template.

10. pR2C6

The pUC19 vector plasmid was treated with the restriction enzymes (AatII and PciI). The following three fragments were inserted therein using In-Fusion. Primer sequence 5'-gaaaagtgccacctgacgtcgcggccgcggaggggtggagt cgtgacgtgaattacgtcat agggttagggaggtcctgtattagaggtcacgtgagtgttttgcgac-3' (SEQ ID NO: 15), 5'-gttcaaacctcccgcttcaaa atggagaccctgcgtgctcactcgggcttaaatacccagc gtgaccacatggtgtcgcaaaatgtcgcaaaacactca-3' (SEQ ID NO: 16), no template. Primer sequences 5'-gcgggaggtttgaacgcgcagccgccATGCCGGGGTTTTAC-3' (SEQ ID NO: 17) and 5'-actagtA GCGCTatttaaatcatTTATTGTTCAAAGAT-3' (SEQ ID NO: 18) were used with pRC2-mi342 (Takara as a template. Primer sequences 5'-aatAGCGCTGCGATCGCggcctccaaaaaagc-3' (SEQ ID NO: 19) and 5'-ccttttgctcacatgtcaattgatcccgcccc taact-3' (SEQ ID NO: 20) were used with the SV40ori gene sequence (TOYOBO) as a template. The obtained plasmid was treated with the restriction enzymes (SwaI and AfeI). The following fragments were inserted therein using In-Fusion. Primer sequences 5'-AACAATAAatgatttaaatcagg-3' (SEQ ID NO: 21) and 5'-ggccGCGATCGCAGCGCTgtagccatggaa actagata-3' (SEQ ID NO: 22) were used with an artificially synthesized AAV6 Cap gene as a template. The obtained plasmid (pR2C6_1) was treated with the restriction enzyme (NotI). The following fragments were inserted therein using In-Fusion. Primer sequence 5'-gtgccacctgacgtcAAATAATGATTTTATTTTGACTGATAGTGACCTGTTCGTTGCAACA AATTGATGAGCAAT-3' (SEQ ID NO: 23), 5'-cgactccacccctccgcggccgcCAACTTTATTATACAAAGTTGGCATTATAAAA AAGCAT TGCTCATCAATTT-3' (SEQ ID NO: 24), no template. The obtained plasmid was treated with the restriction enzyme (AsiSI). The following fragments were inserted therein using In-Fusion. Primer sequence 5'-catggctacAGCG CTGCGATCGCACCCAGCTTTCTTGTACAAAGTTGGCATTATAAGAAAG CATTGCTTATCAATTTG-3' (SEQ ID NO: 25), 5'-tttttggaggccGCGCAAATAATGATTTTATTTTGACTGATAGTGACCTGTTCGTTGCAAC AAATTGATAAGCAA-3' (SEQ ID NO: 26), no template.

11. pR3C6

The above 10.pR2C6 (plasmid #10) was treated with the restriction enzymes (SwaI and NotI). The following fragments were inserted therein using In-Fusion. Primer sequences 5'-ATAAAGTTGgcggccgcagtgacgtaacgcgaagc-3' (SEQ ID NO: 27) and 5'-AGCCATacctgatttaaatcatTTATTGCTCAGAA-3' (SEQ ID NO: 28) were used with an artificially synthesized AAV3 Rep gene as a template.

12. pR5C6

The above 10.pR2C6 (plasmid #10) was treated with the restriction enzymes (SwaI and NotI). The following fragments were inserted therein using In-Fusion. Primer sequences 5'-ATAAAGTTGgcggccgcgggttttgtaagcagtga-3' (SEQ ID NO: 29) and 5'-AGCCATacctgatttaaatcatTTACTGTTCTTTA-3' (SEQ ID NO: 30) were used with an artificially synthesized AAV5 Rep gene as a template.

13. pR7C6

The above 10.pR2C6 (plasmid #10) was treated with the restriction enzymes (SwaI and NotI). The following fragments were inserted therein using In-Fusion. Primer sequences 5'-ATAAAGTTGgcggccgcaagcgcctcccacgctg-3' (SEQ ID NO: 31) and 5'-AGCCATacctgatttaaatcatTTATTGCTCAGAA-3' (SEQ ID NO: 32) were used with an artificially synthesized AAV7 Rep gene as a template.

14. pR8C6

The above 10.pR2C6 (plasmid #10) was treated with the restriction enzymes (SwaI and NotI). The following fragments were inserted therein using In-Fusion. Primer sequences 5'-ATAAAGTTGgcggccgccagagagggagtggccaa-3' (SEQ ID NO: 33) and 5'-AGCCATacctgatttaaatcatTTATTGCTCAGAA-3' (SEQ ID NO: 34) were used with an artificially synthesized AAV8 Rep gene as a template.

15. pR10C6

The above 10.pR2C6 (plasmid #10) was treated with the restriction enzymes (SwaI and NotI). The following fragments were inserted therein using In-Fusion. Primer sequences 5'-ATAAAGTTGgcggccgccagagagggagtggccaa-3' (SEQ ID NO: 35) and 5'-AGCCATacctgatttaaatcatTTATTGCTCAGAA-3' (SEQ ID NO: 36) were used with an artificially synthesized AAV10 Rep gene as a template.

16. pR2C9

pR2C6_1 was treated with the restriction enzymes (SwaI and AfeI). The following fragments were inserted therein using In-Fusion. Primer sequences 5'-AACAATAAatgatttaaatcagg-3' (SEQ ID NO: 37) and 5'-ggccGCGATCG CAGCGCTgtagccatggaaactagata-3' (SEQ ID NO: 38) were used with an artificially synthesized AAV9 Cap gene as a template.

17. pAAV-ITR3-EGFP

It was produced by gene synthesis and the OGAB method. The genes contained in this plasmid and their order are as follows: 5' ITR3 (artificial gene synthesis), CMV promoter (TAKARA), EGFP (VectorBuilder), hGH polyA signal (TAKARA), 3' ITR3 (artificial gene synthesis), ampicillin resistance gene (pUC19), and *E. coli* origin of replication (pUC19). Here, an exemplary procedure of the OGAB method is shown below. The target nucleic acid to be introduced was divided into fragments of about 1 kb or less. The ends of each fragment were designed to be unique and complementary only to a specific fragment. Primers were designed for each of the fragments thus designed, and each fragment was prepared by PCR. Each fragment was cloned into a vector plasmid, and transformation of *E. coli* was performed using each vector plasmid to obtain clones containing the correct sequence for each fragment. Plasmids were purified from these *E. coli* clones, and the DNA concentration of the plasmid solutions was measured. The respective plasmid solutions were aliquoted and mixed so that the amounts of the plasmids of the respective fragments were equal. A restriction enzyme was added to the mixed solution to generate DNA fragments each having an end that is unique and complementary only to a specific fragment. Thereafter, an OGAB assembly vector cleaved with the same restriction enzyme was added to the solution in an amount adjusted to match the molar concentration of each fragment, and ligation was performed to link pairs of fragments (including fragments of the OGAB assembly vector) having complementary ends. This was added to *Bacillus subtilis* competent cells and cultured, and then, transformed colonies were obtained. A plasmid having the desired structure was recovered from this colony.

(Production of rAAV (293T cells)) A schematic diagram of the construct is illustrated in FIG. 1.

The day before transfection, 6 x $10^6$ 293T cells were seeded in a 10 cm-dish. After 22 hours, the medium was replaced with 9.5 mL of DMEM medium (2% FBS, 1% penicillin/streptomycin), and culture was performed at 37°C with 5% $CO_2$ for 2 hours. A transfection solution was added to the 293T cells in a confluent state, the transfection solution obtained by mixing 250 μL of DMEM medium containing each of 49 types of plasmid mixed solutions, each prepared by combining one type of pHelper, seven types of pAAV, and seven types of pRC (FIG. 1) and 250 μL of DMEM medium containing PEIpro (registered trademark) (PolyPlus-transfection SAS) in an amount equal to the amount of DNA.

[Table 2]

Table 2: Plasmid DNA used for 293T cells

| pDNA# | Transfected pDNA | Length [bp] | Transfected pDNA amount [μg] |
|---|---|---|---|
| 1 | pHelper | 11120 | 8.91 |
| 2 | pAAV-ITR1-EGFP | 4136 | 3.31 |
| 3 | pAAV-ITR2wt-EGFP | 4140 | 3.32 |
| 17 | pAAV-ITR3-EGFP | 4724 | 3.78 |
| 4 | pAAV-ITR4-EGFP | 4122 | 3.30 |
| 5 | pAAV-ITR5-EGFP | 4843 | 3.88 |
| 6 | pAAV-ITR6-EGFP | 4132 | 3.31 |
| 7 | pAAV-ITR7-EGFP | 4144 | 3.32 |
| 8 | pAAV-ITR2 mutant -EGFP | 5245 | 4.20 |
| 9 | pR1C6 | 6557 | 5.25 |
| 10 | pR2C6 | 6535 | 5.24 |
| 11 | pR3C6 | 6540 | 5.24 |
| 12 | pR5C6 | 6528 | 5.23 |
| 13 | pR7C6 | 6552 | 5.25 |
| 14 | pR8C6 | 6598 | 5.29 |

(continued)

| Table 2: Plasmid DNA used for 293T cells | | | |
|---|---|---|---|
| pDNA# | Transfected pDNA | Length [bp] | Transfected pDNA amount [μg] |
| 15 | pR10C6 | 6589 | 5.28 |

The cells were cultured at 37°C with 5% $CO_2$ for 72 hours and then suspended in 1 mL of 10 × Lysis buffer (20 mM $MgCl_2$, 1.5 M NaCl, 500 mM Tris, 1% Triton (registered trademark) X-100 (NACALAI TESQUE, INC.), pH7.5), 0.5 μL of Benzonase (registered trademark) (Novagen) was further added, and the mixture was reacted at 37°C for 60 minutes. Thereafter, 2/100 volume of a 1.9 M $MgSO_4$ solution was added, and the mixture was allowed to stand at room temperature for 10 minutes. The supernatant was collected by centrifugation at 12,000 x g and 4°C for 10 minutes and Pluronic (registered trademark) F-68 (Thermo Fisher Scientific) was added at a final concentration of 0.001%.

Density gradient centrifugation was performed by layering three types of cesium chloride solutions (1.35 g/$cm^3$, 1.27 g/$cm^3$, and 1.25 g/$cm^3$) and 8 mL of the sample solution, at 226,000 × g, 18°C for 1.5 hours, and about 2 mL of rAAV was collected from the solution around 1.27 g/$cm^3$. Pluronic (registered trademark) F-68 (Thermo Fisher Scientific) was added at a final concentration of 0.001%. Furthermore, buffer exchange was performed using 100 K Amicon Ultra-4 with a buffer (10% sucrose, 100 mM Tris, 200 mM NaCl, 0.005% Pluronic (registered trademark) F-68, pH 7.4), and the solution was concentrated to about 0.5 mL.

(Production of rAAV (293 cells))

A schematic diagram of the construct is illustrated in FIG. 3.

The day before transfection, 3.62 x $10^6$ 293 cells were seeded in a 10 cm-dish. After 22 hours, the medium was replaced with 9.5 mL of DMEM medium (2% FBS, 1% penicillin/streptomycin), and culture was performed at 37°C with 5% $CO_2$ for 2 hours. A transfection solution was added to the 293 cells in a confluent state, the transfection solution being obtained by mixing 250 μL of DMEM medium containing each of 14 types of plasmid mixed solutions, each prepared by combining one type of pHelper, seven types of pAAV, and two types of pRC (FIG. 2) and 250 μL of DMEM medium containing PEIpro (registered trademark) (PolyPlus-transfection SAS) in an amount equal to the amount of DNA.

[Table 3]

| Table 3: Plasmid DNA used for 293 cells | | | |
|---|---|---|---|
| pDNA# | Transfected pDNA | Length [bp] | Transfected pDNA amount [μg] |
| 1 | pHelper | 11120 | 3.75 |
| 2 | pAAV-ITR1-EGFP | 4136 | 1.39 |
| 3 | pAAV-ITR2wt-EGFP | 4140 | 1.40 |
| 17 | pAAV-ITR3-EGFP | 4724 | 1.59 |
| 4 | pAAV-ITR4-EGFP | 4122 | 1.39 |
| 5 | pAAV-ITR5-EGFP | 4843 | 1.63 |
| 6 | pAAV-ITR6-EGFP | 4132 | 1.39 |
| 7 | pAAV-ITR7-EGFP | 4144 | 1.40 |
| 8 | pAAV-ITR2 mutant-EGFP | 5245 | 1.77 |
| 10 | pR2C6 | 6535 | 2.20 |
| 16 | pR2C9 | 6336 | 2.14 |

The cells were cultured at 37°C with 5% $CO_2$ for 72 hours and then suspended in 1 mL of 10 × Lysis buffer (20 mM $MgCl_2$, 1.5 M NaCl, 500 mM Tris, 1% Triton (registered trademark) X-100 (NACALAI TESQUE, INC.), pH7.5), 0.5 μL of Benzonase (registered trademark) (Novagen) was further added, and the mixture was reacted at 37°C for 60 minutes. Thereafter, 2/100 volume of a 1.9 M $MgSO_4$ solution was added, and the mixture was allowed to stand at room temperature for 10 minutes. The supernatant was collected by centrifugation at 12,000 x g and 4°C for 10 minutes and Pluronic (registered trademark) F-68 (Thermo Fisher Scientific) was added at a final concentration of 0.001%.

Density gradient centrifugation was performed by layering three types of cesium chloride solutions (1.35 g/$cm^3$,

1.27 g/cm$^3$, and 1.25 g/cm$^3$) and 8 mL of the sample solution, at 226,000 × g, 18°C for 1.5 hours, and about 2 mL of rAAV was collected from the solution around 1.27 g/cm$^3$. Pluronic (registered trademark) F-68 (Thermo Fisher Scientific) was added at a final concentration of 0.001%. Furthermore, buffer exchange was performed using 100 K Amicon Ultra-4 with a buffer (10% sucrose, 100 mM Tris, 200 mM NaCl, 0.005% Pluronic (registered trademark) F-68, pH 7.4), and the solution was concentrated to about 0.5 mL.

(Evaluation of rAAV)

In order to quantify the genome titer of rAAV, the sample was treated with DNase I for 30 minutes, reacted at 95°C for 10 minutes, and then diluted 1,000-fold for qPCR. Primer sequences 5'-CATCAATGGGCGTGGATAGC-3' (SEQ ID NO: 39) and 5'-GGAGTTGTTACGACATTTTGGAAA-3' (SEQ ID NO: 40) targeting the CMV promoter, were used. As PCR conditions, after 10 minutes at 95°C, 40 cycles of 95°C/15 seconds, 55°C/5 seconds, and 72°C/30 seconds were repeated.

Quantitative analysis by HPLC was performed to determine a full ratio. A fluorometer was used as a detector, and CIMac AAV full/empty column-0.1 (inner diameter: 5.2 mm, length: 4.95 mm, channel diameter: 1.3 μm, BIA Separations) was used as a column. The mobile phases used were: A: ultrapure water, B: 1 M tetramethylammonium chloride solution, and C: 250 mM bis-tris propane (pH 9.0), these mobile phases were delivered at a flow rate of 0.5 mL/min, and a concentration gradient was controlled by varying the mixing ratios as shown in Table 3 for AAV6 and Table 4 for AAV9. 5 μL of the sample produced using 293T cells and 25 μL of the sample produced using 293 cells were injected.

[Table 4]

| Table 4: HPLC gradient program (for AAV6) | | | |
|---|---|---|---|
| Time after injection (min) | Mobile phase A (vol%) | Mobile phase B (vol%) | Mobile phase C (vol%) |
| 0 | 72 | 0 | 28 |
| 25 | 47 | 25 | 28 |
| 30 | 0 | 72 | 28 |

[Table 5]

| Table 5: HPLC gradient program (for AAV9) | | | |
|---|---|---|---|
| Time after injection (min) | Mobile phase A (vol%) | Mobile phase B (vol%) | Mobile phase C (vol%) |
| 0 | 72 | 0 | 28 |
| 0.5 | 72 | 0 | 28 |
| 3.5 | 69 | 3 | 28 |
| 8 | 47 | 25 | 28 |
| 13 | 0 | 72 | 28 |
| 13.1 | 72 | 0 | 28 |
| 17 | 72 | 0 | 28 |

(Results)

The results are illustrated in FIGS. 2 and 4. As shown, the yield of AAV vectors (titer and full ratio) was highly dependent on the combination of Rep and ITR. Rep2 exhibited high titer and full ratio in combination with any of the ITRs, suggesting that it interacts with all of them. Rep1, 7, and 10 exhibited high titer and full ratio in combination with ITRs other than ITR5. Rep8 exhibited low titer and full ratio in combination with any ITR.

(Example 2: Calculation of free energy)

(Methods)

The base sequence of the ITR is input into the DNA Folding Form of the Mfold web server (http://www.unafold.org/mfold/applications/dna-folding-form.php), and Fold DNA is executed. (Default settings were used)

(Results)

The results are illustrated in FIG. 5. When the results of Example 1 are also taken into consideration, the change in free energy ($\Delta G$) of the ITR may be in a range of - 65 to -95 kcal/mol, preferably -75 to -95 kcal/mol, more preferably -80 to -90 kcal/mol, and most preferably -80 to - 88 kcal/mol.

(Example 3: Example of other Reps)

In the present example, it is demonstrated that the desired effect is exhibited even when the Rep sequence is derived from any AAV of the serotype 1/6 family, serotype 2 family, serotype 3/4 family, serotype 12 family, serotype 5 family, serotype 8 family, serotype 7/9 family, or serotype 10/11 family.
In the present example, an experiment similar to that of Example 1 was conducted, in which the plasmid DNA used in 293T cells shown in Table 2 and the transfected pDNA were replaced with pR4C6, pR6C6, and pR9C6. Each base length is 6,541 bp.
Rep4, Rep6, and Rep9 are expected to exhibit the similar titer and full ratio to those of Rep3, Rep1, and Rep7, respectively.

(Note)

As described above, the present disclosure is exemplified by the use of its preferred embodiments. It is understood that the scope of the present disclosure should be interpreted solely based on the Claims. It is also understood that any patent, any patent application, and any references cited herein should be incorporated herein by reference in the same manner as the contents are specifically described herein. The present application claims priority to Japanese Patent Application No. 2023-023545, filed on February 17, 2023 to the Japan Patent Office, the entire contents of which are incorporated herein by reference.

Industrial Applicability

The present disclosure provides plasmid DNA or a DNA/cationic substance complex containing a Rep and an ITR and exhibiting high AAV vector productivity.

Sequence Listing Free Text

SEQ ID NO: 1: CMV-F primer (CATCAATGGGCGTGGATAGC)
SEQ ID NO: 2: CMV-R primer (GGAGTTGTTACGACATTTTGGAAA)
SEQ ID NO: 3: Primer sequence 5'-ttagtaagcttccctcctgacgcggtaggaggaggggagggtgccctgcatg-3',
SEQ ID NO: 4: Primer sequence5'-ccttttgctcacatgtcaattggtagatgtacctggacatccaggtgatgccggcg-3'
SEQ ID NO: 5: Primer sequence5'-

tgtacaGGCGCGCCgaattcgttttagggcggagtaacttgtatgtgttgggaattgtag-3'

SEQ ID NO: 6: Primer sequence5'-agggaagcttactaaacggtacacaggaaacaggagacacaactccaagtg-3'
SEQ ID NO: 7: Primer sequence5'-

gaaaagtgccacctgacgtcgcggccgcGCGATCGCggtacccaactccatgctcaacagtccccaggtacagccc-3'

SEQ ID NO: 8: Primer sequence5'-

gaattcGGCGCGCCtgtacagcccgggcgaccgcaccctgtgacgaaagccgcccgcaag-3'

SEQ ID NO: 9: Primer sequence5'-

cgaaaagtgccacctAAATAATGATTTTATTTTGACTGATAGTGACCTGTTCGTTGCAACA AATTGATGAGCAA-3'

SEQ ID NO: 10: Primer sequence5'-

caggccCACCTGCtcagattaCAACTTTTGTATACAAAGTTGGCATTATAAAAAAGCATTG CTCATCAATTTG-3'

SEQ ID NO: 11: Primer sequence5'- gccCGAATCCCGGCCGGCCACCATGGTGAGCAAG-3'
SEQ ID NO: 12: Primer sequence5'-TGCCACCCGTggatccTTACTTGTACAGCTCGTC-3'
SEQ ID NO: 13: Primer sequence5'-ATAAAGTTGgcggccgctcgcgaagcgcctcccac-3'
SEQ ID NO: 14: Primer sequence5'-AGCCATacctgatttaaatcatTTATTGCTCAGAA-3'
SEQ ID NO: 15: Primer sequence5'-

gaaaagtgccacctgacgtcgcggccgcggaggggtggagtcgtgacgtgaattacgtcat agggttagggaggtcctgtattagaggtcacgtgagtgttttgcgac-3'

SEQ ID NO: 16: Primer sequence5'-

gttcaaacctcccgcttcaaaatggagaccctgcgtgctcactcgggcttaaatacccagc gtgaccacatggtgtcgcaaaatgtcgcaaaacactca-3'

SEQ ID NO: 17: Primer sequence5'-gcgggaggtttgaacgcgcagccgccATGCCGGGGTTTTAC-3'
SEQ ID NO: 18:5'-actagtAGCGCTatttaaatcatTTATTGTTCAAAGAT-3'
SEQ ID NO: 19: Primer sequence5'-aatAGCGCTGCGATCGCggcctccaaaaaagc-3'
SEQ ID NO: 20: Primer sequence5'-ccttttgctcacatgtcaattgatcccgcccctaact-3'
SEQ ID NO: 21: Primer sequence5'-AACAATAAatgatttaaatcagg-3'
SEQ ID NO: 22: Primer sequence5'-ggccGCGATCGCAGCGCTgtagccatggaaactagata-3'
SEQ ID NO: 23: Primer sequence5'-

gtgccacctgacgtcAAATAATGATTTTATTTTGACTGATAGTGACCTGTTCGTTGCAACA AATTGATGAGCAAT-3'

SEQ ID NO: 24: Primer sequence5'-

cgactccacccctccgcggccgcCAACTTTATTATACAAAGTTGGCATTATAAAAAAGCAT TGCTCATCAATTT-3'

SEQ ID NO: 25: Primer sequence5'-

catggctacAGCGCTGCGATCGCACCCAGCTTTCTTGTACAAAGTTGGCATTATAAGAAAG CATTGCTTATCAATTTG-3'

SEQ ID NO: 26: Primer sequence5'-

tttttggaggccGCGCAAATAATGATTTTATTTTGACTGATAGTGACCTGTTCGTTGCAAC AAATTGATAAGCAA-3'

SEQ ID NO: 27: Primer sequence5'-ATAAAGTTGgcggccgcagtgacgtaacgcgaagc-3'
SEQ ID NO: 28: Primer sequence5'-AGCCATacctgatttaaatcatTTATTGCTCAGAA-3'
SEQ ID NO: 29: Primer sequence5'-ATAAAGTTGgcggccgcgggttttgtaagcagtga-3'
SEQ ID NO: 30: Primer sequence5'-AGCCATacctgatttaaatcatTTACTGTTCTTTA-3'
SEQ ID NO: 31: Primer sequence5'-ATAAAGTTGgcggccgcaagcgcctcccacgctg-3'

SEQ ID NO: 32: Primer sequence5'-AGCCATacctgatttaaatcatTTATTGCTCAGAA-3'
SEQ ID NO: 33: Primer sequence5'-ATAAAGTTGgcggccgccagagagggagtggccaa-3'
SEQ ID NO: 34: Primer sequence5'-AGCCATacctgatttaaatcatTTATTGCTCAGAA-3'
SEQ ID NO: 35: Primer sequence5'-ATAAAGTTGgcggccgccagagagggagtggccaa-3'
SEQ ID NO: 36: Primer sequence5'-AGCCATacctgatttaaatcatTTATTGCTCAGAA-3'
SEQ ID NO: 37: Primer sequence5'-AACAATAAatgatttaaatcagg-3'
SEQ ID NO: 38: Primer sequence5'-ggccGCGATCGCAGCGCTgtagccatggaaactagata-3'
SEQ ID NO: 39: Primer sequence5'-CATCAATGGGCGTGGATAGC-3'
SEQ ID NO: 40: Primer sequence5'-GGAGTTGTTACGACATTTTGGAAA-3'

Sequence Listing

International application No. 093459 under the International Patent Cooperation Treaty ITR-Rep Gene Complex JP24004838 20240213----00100110952400317440 Normal 20240213153335202401241354048900_P1AP101_09_5.xml

## Claims

1. A nucleic acid comprising:

   a Rep gene derived from an adeno-associated virus (AAV) of a first serotype; and
   an inverted terminal repeat (ITR) sequence derived from an AAV of a second serotype different from the first serotype and having a ΔG value in a range of -65 to -95 kcal/mol.

2. The nucleic acid according to claim 1, wherein the Rep gene is derived from an AAV of serotype 2, and the ITR sequence has a ΔG value in a range of -75 to -95 kcal/mol.

3. The nucleic acid according to claim 2, wherein the ITR sequence has a ΔG value in a range of -80 to -88 kcal/mol.

4. The nucleic acid according to claim 2 or 3,
wherein the ITR sequence is derived from an AAV of serotype 1, 6, or 7.

5. The nucleic acid according to claim 1, wherein the Rep gene is derived from an AAV of serotype 1, 2, 5, 7, or 10, and the ITR sequence has a ΔG value in a range of -75 to -95 kcal/mol.

6. The nucleic acid according to claim 5, wherein the ITR sequence has a ΔG value in a range of -80 to -90 kcal/mol.

7. The nucleic acid according to claim 5 or 6,
wherein the ITR sequence is derived from an AAV of any one of serotypes 1 to 7.

8. The nucleic acid according to any one of claims 1 to 7, further comprising a Cap gene derived from an AAV.

9. The nucleic acid according to any one of claims 1 to 7, further comprising a Cap gene derived from an AAV of any one of serotypes 1 to 10.

10. The nucleic acid according to any one of claims 1 to 9, wherein the Rep gene, the ITR sequence, and a Cap gene derived from an AAV are contained on the same nucleic acid molecule or nucleic acid duplex.

11. The nucleic acid according to any one of claims 1 to 10, further comprising a helper gene derived from an adenovirus (AdV).

12. The nucleic acid according to any one of claims 1 to 11, wherein a combination of the Rep gene and the ITR sequence is a combination of the Rep gene and the ITR sequence, which has an ability to produce a viral vector with a titer [GC/mL] that is twice or more than that of a combination of the said Rep gene and ITR-5, when measured by qPCR targeting a CMV promoter or EGFP.

13. The nucleic acid according to any one of claims 1 to 12, wherein the nucleic acid is a plasmid.

**14.** The nucleic acid according to any one of claims 1 to 13, wherein the nucleic acid forms a complex with a cationic substance.

**15.** The nucleic acid according to claim 14, wherein the cationic substance contains a polymer or a liposome.

**16.** A composition for producing a viral vector, the composition comprising the nucleic acid according to any one of claims 1 to 15.

**17.** A method for synthesizing the nucleic acid according to any one of claims 1 to 16, the method comprising a step of combining a nucleic acid containing the Rep gene and a nucleic acid containing the ITR.

**18.** A method for producing a viral vector, the method comprising:

a step of introducing the nucleic acid according to any one of claims 1 to 15 or the composition according to claim 16 into a producer cell; and
a step of forming a viral vector in the producer cell.

**19.** The method according to claim 18, wherein at least a part of the nucleic acid is integrated into a chromosome of the producer cell.

**20.** A producer cell or a viral vector produced by the method according to claim 18 or 19.

[Fig. 1]

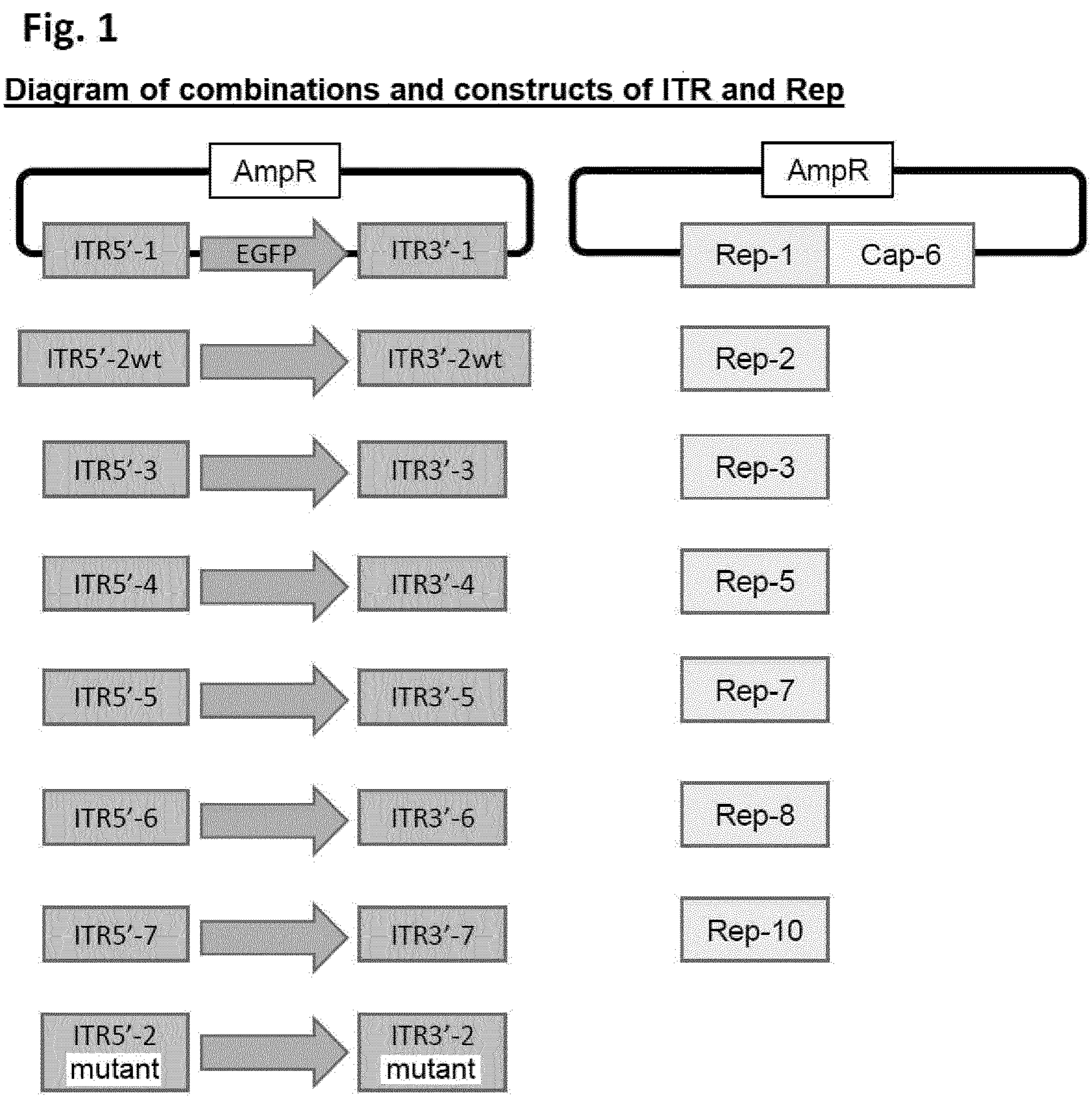
Fig. 1
Diagram of combinations and constructs of ITR and Rep
AmpR
ITR5'-1
EGFP
ITR3'-1
AmpR
Rep-1
Cap-6
ITR5'-2wt
ITR3'-2wt
Rep-2
ITR5'-3
ITR3'-3
Rep-3
ITR5'-4
ITR3'-4
Rep-5
ITR5'-5
ITR3'-5
Rep-7
ITR5'-6
ITR3'-6
Rep-8
ITR5'-7
ITR3'-7
Rep-10
ITR5'-2 mutant
ITR3'-2 mutant

[Fig. 2]

**Fig. 2**

qPCR (GC/mL)

| | Rep-1 | Rep-2 | Rep-3 | Rep-5 | Rep-7 | Rep-8 | Rep-10 |
|---|---|---|---|---|---|---|---|
| ITR-1 | 5.52E+11 | 3.03E+12 | 4.10E+09 | 9.06E+08 | 9.97E+11 | 1.21E+09 | 6.34E+11 |
| ITR-2wt | 9.45E+11 | 2.79E+12 | 7.20E+09 | 9.94E+08 | 6.35E+11 | 1.05E+09 | 6.83E+11 |
| ITR-3 | 2.05E+11 | 5.42E+11 | 1.40E+10 | 1.38E+07 | 2.29E+11 | 6.24E+06 | 1.74E+11 |
| ITR-4 | 3.31E+11 | 1.25E+12 | 1.40E+09 | 8.96E+08 | 3.69E+11 | 1.00E+09 | 3.30E+11 |
| ITR-5 | 6.95E+10 | 5.03E+11 | 2.25E+09 | 1.04E+12 | 9.54E+10 | 1.19E+09 | 3.69E+10 |
| ITR-6 | 1.25E+12 | 3.60E+12 | 3.05E+10 | 9.52E+08 | 1.64E+12 | 1.25E+09 | 9.23E+11 |
| ITR-7 | 1.01E+12 | 2.70E+12 | 6.14E+10 | 1.06E+09 | 1.09E+12 | 1.23E+09 | 1.20E+12 |
| ITR-2 mutant | 4.67E+11 | 2.22E+12 | 2.34E+10 | 1.09E+09 | 6.27E+11 | 1.40E+09 | 4.08E+11 |

HPLC (Full ratio %)

| | Rep-1 | Rep-2 | Rep-3 | Rep-5 | Rep-7 | Rep-8 | Rep-10 |
|---|---|---|---|---|---|---|---|
| ITR-1 | 90.6 | 92.8 | 11.2 | 11.0 | 76.1 | N.D. | 80.4 |
| ITR-2wt | 87.0 | 91.5 | 12.3 | 11.6 | 83.6 | N.D. | 87.9 |
| ITR-3 | 91.3 | 88.0 | 10.7 | 7.8 | 81.3 | N.D. | 87.9 |
| ITR-4 | 84.8 | 68.8 | 31.1 | 24.8 | 79.1 | N.D. | 86.2 |
| ITR-5 | N.D. | 81.6 | N.D. | 87.6 | 27.3 | N.D. | 33.7 |
| ITR-6 | 92.0 | 92.3 | 30.9 | 22.0 | 91.1 | N.D. | 91.1 |
| ITR-7 | 90.3 | 93.9 | 12.6 | 13.9 | 86.5 | N.D. | 84.6 |
| ITR-2 mutant | 96.2 | 96.9 | 22.0 | N.D. | 95.6 | N.D. | 98.5 |

[Fig. 3]

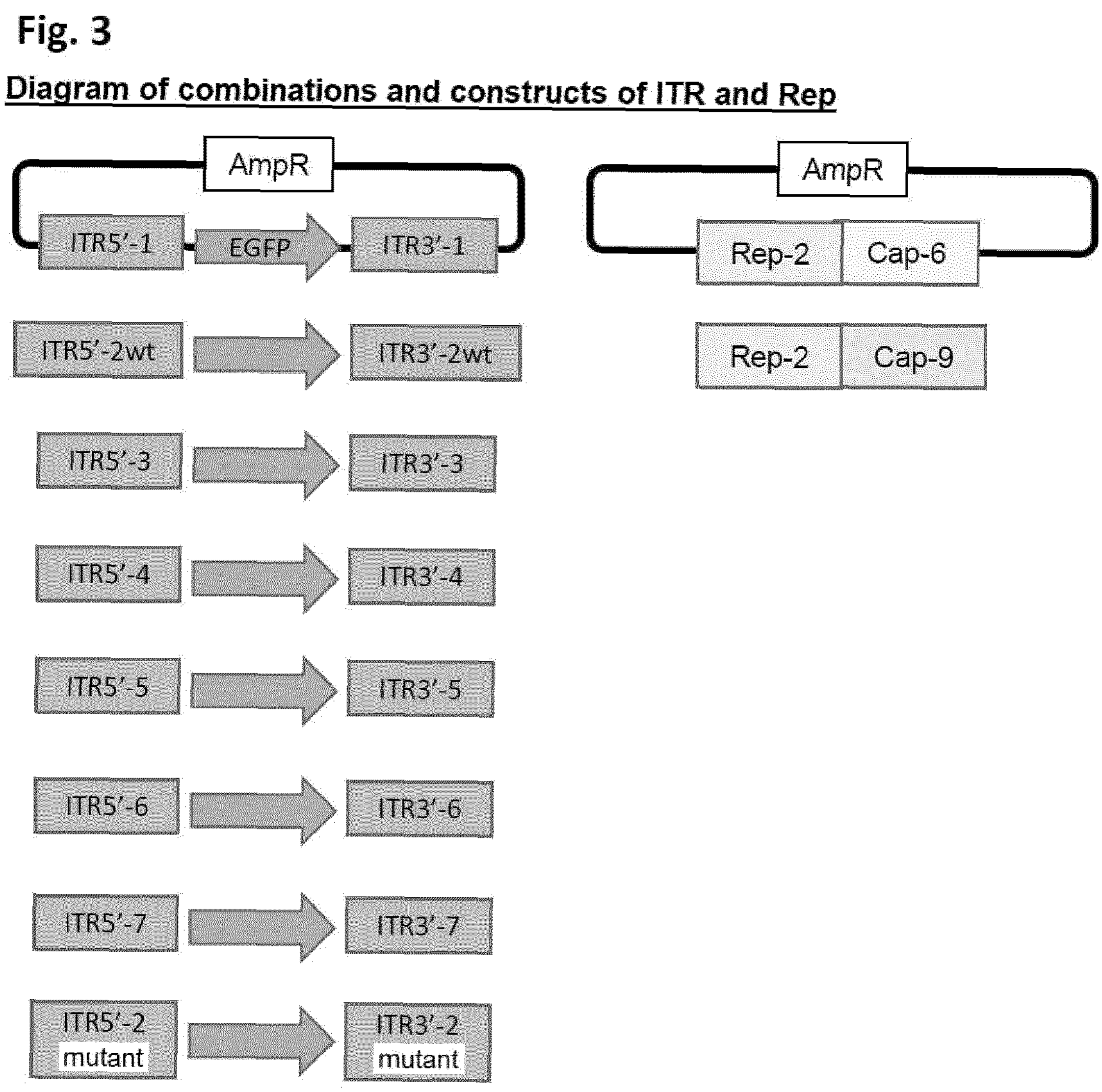
Fig. 3
Diagram of combinations and constructs of ITR and Rep
AmpR
ITR5'-1
EGFP
ITR3'-1
ITR5'-2wt
ITR3'-2wt
ITR5'-3
ITR3'-3
ITR5'-4
ITR3'-4
ITR5'-5
ITR3'-5
ITR5'-6
ITR3'-6
ITR5'-7
ITR3'-7
ITR5'-2 mutant
ITR3'-2 mutant
AmpR
Rep-2
Cap-6
Rep-2
Cap-9

[Fig. 4]

Fig. 4

qPCR (GC/mL)

| | 293T | 293 | 293 |
|---|---|---|---|
| | Cap6 | Cap6 | Cap9 |
| | Rep-2 | Rep-2 | Rep-2 |
| ITR-1 | 3.03E+12 | 2.13E+11 | 1.44E+11 |
| ITR-2wt | 2.79E+12 | 1.52E+11 | 1.18E+11 |
| ITR-3 | 5.42E+11 | 8.04E+10 | 1.41E+11 |
| ITR-4 | 1.25E+12 | 1.08E+11 | 1.38E+11 |
| ITR-5 | 5.03E+11 | 1.36E+09 | 6.60E+07 |
| ITR-6 | 3.60E+12 | 1.57E+11 | 1.45E+11 |
| ITR-7 | 2.70E+12 | 1.49E+11 | 1.83E+11 |
| ITR-2 mutant | 2.22E+12 | 1.48E+11 | 7.52E+10 |

HPLC (Full ratio %)

| | 293T | 293 | 293 |
|---|---|---|---|
| | Cap6 | Cap6 | Cap9 |
| | Rep-2 | Rep-2 | Rep-2 |
| ITR-1 | 92.8 | 74.8 | 46.8 |
| ITR-2wt | 91.5 | 76.7 | 45.6 |
| ITR-3 | 88.0 | 84.2 | 32.2 |
| ITR-4 | 68.8 | 90.4 | 39.7 |
| ITR-5 | 81.6 | 46.6 | 17.0 |
| ITR-6 | 92.3 | 95.8 | 45.6 |
| ITR-7 | 93.9 | 96.8 | 48.4 |
| ITR-2 mutant | 96.9 | 78.8 | 48.1 |

[Fig. 5]

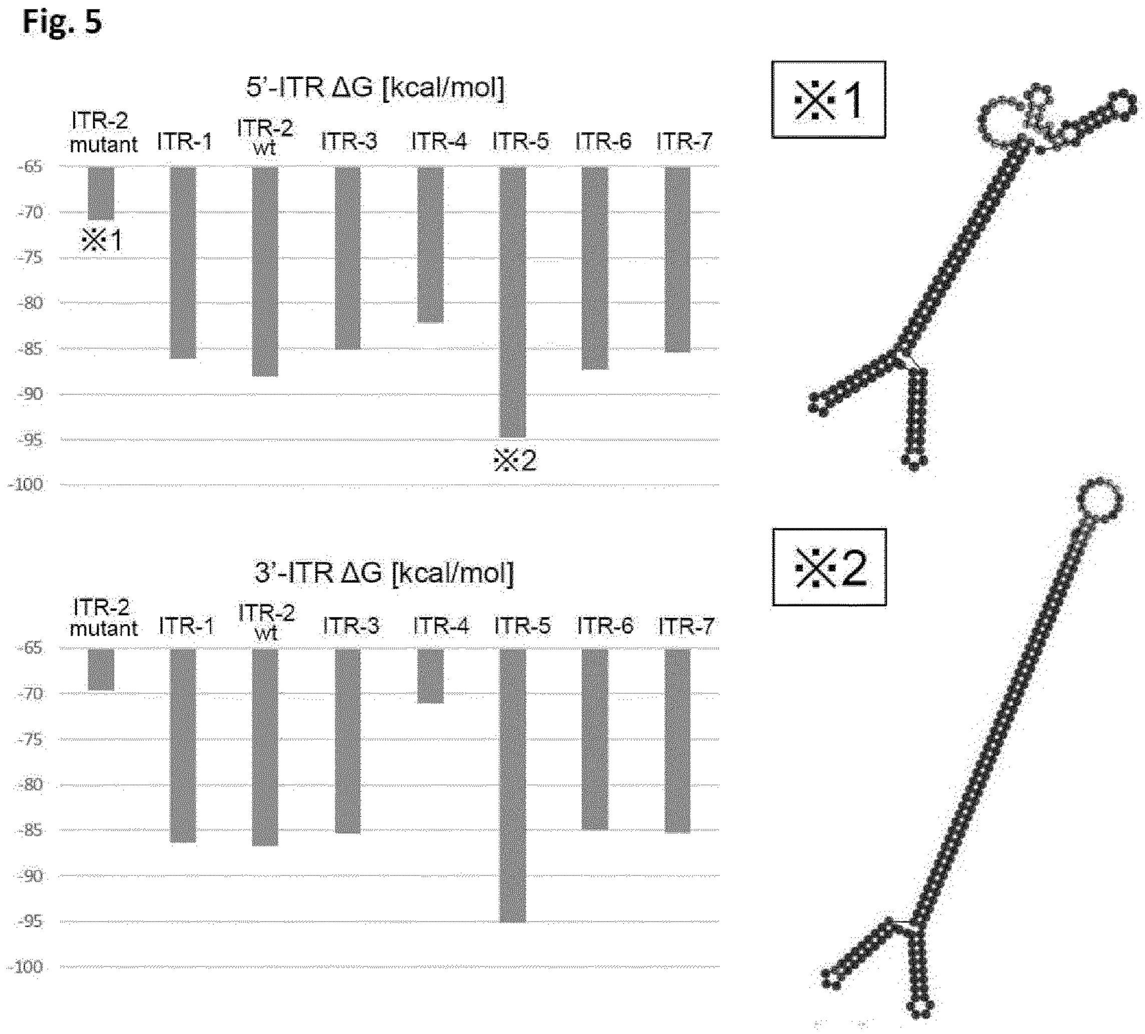
Fig. 5
5'-ITR ΔG [kcal/mol]
ITR-2 mutant
ITR-1
ITR-2 wt
ITR-3
ITR-4
ITR-5
ITR-6
ITR-7
-65
-70
-75
-80
-85
-90
-95
-100
※1
※2
3'-ITR ΔG [kcal/mol]
ITR-2 mutant
ITR-1
ITR-2 wt
ITR-3
ITR-4
ITR-5
ITR-6
ITR-7
-65
-70
-75
-80
-85
-90
-95
-100
※1
※2

**INTERNATIONAL SEARCH REPORT**

International application No.
**PCT/JP2024/004838**

**A. CLASSIFICATION OF SUBJECT MATTER**

***C12N 15/864***(2006.01)i; ***C12N 15/34***(2006.01)i; ***C12N 15/35***(2006.01)i; ***C12N 15/88***(2006.01)i
FI: C12N15/864 100Z; C12N15/34; C12N15/35 ZNA; C12N15/88 Z

According to International Patent Classification (IPC) or to both national classification and IPC

**B. FIELDS SEARCHED**

Minimum documentation searched (classification system followed by classification symbols)

C12N15/864; C12N15/34; C12N15/35; C12N15/88

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2021-514659 A (UNIVERSITY OF FLORIDA RESEARCH FOUNDATION, INCORPORATED) 17 June 2021 (2021-06-17)<br>claims 1-2, 4, 15-16, 22-23, 60, paragraphs [0073]-[0074], [0076], [0080], [0088], [0096], examples | 1-20 |
| X | WO 2021/142130 A1 (THE UNIVERSITY OF NORTH CAROLINA AT CHAPEL HILL) 15 July 2021 (2021-07-15)<br>claims 20, 22, 25-27, p. 25, lines 6-12, p. 55, line 23 to p. 56, line 7 | 1-20 |
| X | WO 2022/097646 A1 (SYNPLOGEN COMPANY, LIMITED) 12 May 2022 (2022-05-12)<br>claims 1, 13, 18-27, 41, 43-44, example 7, table 6, vetor plasmid no. 6-7, fig. 13 | 1-20 |
| X | WO 2022/097647 A1 (SYNPLOGEN COMPANY, LIMITED) 12 May 2022 (2022-05-12)<br>claims 1-2, 18-19, 23-27, 43-45, example 8, table 6, vetor plasmid no. 6-7, fig. 17 | 1-20 |
| A | WO 2021/146591 A2 (ASKLEPIOS BIOPHARMACEUTICAL, INC.) 22 July 2021 (2021-07-22)<br>entire text, all drawings | 1-20 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 May 2024** | **14 May 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br>Telephone No. |

Form PCT/ISA/210 (second sheet) (July 2022)

**INTERNATIONAL SEARCH REPORT**

International application No.
**PCT/JP2024/004838**

**C. DOCUMENTS CONSIDERED TO BE RELEVANT**

| Category* | Citation of document, with indication, where appropriate, of the relevant passages | Relevant to claim No. |
|---|---|---|
| A | JP 2004-514407 A (THE TRUSTEES OF THE UNIVERSITY OF PENNSYLVANIA) 20 May 2004 (2004-05-20) entire text, all drawings | 1-20 |
| P, X | WO 2023/214579 A1 (SYNPLOGEN COMPANY, LIMITED) 09 November 2023 (2023-11-09) claims 20, 24-27, 30-31, 64-66, example 2, fig. 5 | 1-20 |

Form PCT/ISA/210 (second sheet) (July 2022)

**INTERNATIONAL SEARCH REPORT**

International application No.
**PCT/JP2024/004838**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

**INTERNATIONAL SEARCH REPORT**
**Information on patent family members**

International application No.
**PCT/JP2024/004838**

| Patent document cited in search report | Publication date (day/month/year) | Patent family member(s) | Publication date (day/month/year) |
|---|---|---|---|
| JP 2021-514659 A | 17 June 2021 | WO 2019/173538 A1<br>claims 1-2, 4, 15-16, 22-23, 60, p. 74, lines 7-27, p. 75, lines 1-5, p. 75, lines 25-32, p. 77, table 2, p. 80, lines 8-15, examples<br>US 2020/0407753 A1<br>EP 3762501 A1<br>KR 10-2020-0130337 A<br>CN 112166195 A | |
| WO 2021/142130 A1 | 15 July 2021 | JP 2023-510266 A<br>US 2023/0002786 A1<br>EP 4087918 A1<br>CN 115244174 A | |
| WO 2022/097646 A1 | 12 May 2022 | EP 4242310 A1<br>claims 1, 13, 18-27, 41, 43-44, example 7, table 6 #6-7, fig. 13<br>CN 116438310 A<br>KR 10-2023-0116801 A | |
| WO 2022/097647 A1 | 12 May 2022 | EP 4242316 A1<br>claims 1-2, 18-19, 23-27, 43-45, example 8, table 6 #6-7, fig. 17<br>CN 116391027 A<br>KR 10-2023-0116802 A | |
| WO 2021/146591 A2 | 22 July 2021 | JP 2023-510590 A<br>US 2023/0048994 A1<br>EP 4090750 A2<br>CN 115315518 A | |
| JP 2004-514407 A | 20 May 2004 | WO 2001/083692 A2<br>US 2003/0013189 A1<br>EP 1285078 A2 | |
| WO 2023/214579 A1 | 09 November 2023 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5908845 A
- JP 2023023545 A


### Non-patent literature cited in the description


- **CHIORINI J. A. et al.** *JOURNAL OF VIROLOGY*, 1999
- **P CLOTE**. RNALOSS: a web server for RNA locally optimal secondary structures. *Nucleic Acids Res.*, 01 July 2005, vol. 33, W600-4